# EUROPEAN PATENT APPLICATION

(11) **EP 3 954 761 A1**
(43) Date of publication of application: **16.02.2022**
(21) Application number: 20787633.5
(22) Date of filing: 08.04.2020
(51) Int. Cl.: C12N 5/077

(54) **CHONDROCYTE CULTURE WITH HIGH TISSUE REGENERATION ABILITY**

(30) Priority: 08.04.2019 JP 2019073783
(71) Applicant: GN Corporation Co Ltd, Kofu-shi, Yamanashi 400-0866 (JP); JBM Incorporation, Tokyo 133-0052 (JP)
(72) Inventor: KATO, Shojiro, Tokyo 133-0052 (JP); ABRAHAM, Samuel JK, Kofu-shi Yamanashi 400-0866 (JP); KUBOTA, Sunao, Kunitachi-shi Tokyo 186-0002 (JP)
(74) Representative: Forstmeyer, Dietmar
(86) International application number: PCT/JP2020/015896
(87) International publication number: WO 2020/209316

(57) **Abstract**

The purpose of the present invention is to provide a chondrocyte culture with high tissue regeneration ability. This purpose is met by a method involving a step in which a cell population separated from cartilage tissue is cultured on a thermoreversible polymer.

## Description

### [Technical Field]

The present invention relates to a method of producing a chondrocyte culture, a chondrocyte culture produced by said method, and a therapeutic method using the chondrocyte culture.

### [Background Arts]

Osteoarthritis is characterized by a progressive cartilage damage of joint tissue, caused by various factors including aging, joint damages and obesity, and would particularly be responsible for the joint facility loss and sclerosis in elderly people.

The cartilage tissue of a joint consists of a thin hyaline cartilage which is present on the epiphyseal joint surface. The hyaline cartilage has a tissue structure in which the intercellular space of chondrocytes is fulfilled with Chondrocyte Extracellular Matrix (ECM), which has formed a higher conformation by an interaction between hyaluronic acid-aggrecan network and type-II collagen fiber, etc. Such ECM degradation is greatly involved in cartilage tissue damaging process in OA.

Damage to cartilage tissue will cause the destruction of type-II collagen fibers in ECM, which will then cause release of hyaluronic acid and aggrecan. The released hyaluronic acid and aggrecan will cause apoptosis of chondrocytes and production of interleukin-1, an inflammatory cytokine, which will stimulate production and secretion of ECM-degrading enzymes such as MMP, ADAMTS and HYBID, thereby further accelerating ECM degradation in the cartilage tissue.

Thus, in cartilage tissue in which ECM has been degraded, the elasticity is remarkably reduced and the tissue is embrittled, leading to a vicious cycle in which the tissue will easy be damaged even by a light loading. Moreover, a joint tissue is different from an ordinal tissue in that it receives no blood supply, but instead it exchanges substances in and out of cartilage tissue via diffusion, and there is no cell influx. Therefore, once the tissue homeostasis is destroyed as seen in osteoarthritis, that degenerated state will be maintained, making it difficult to be repaired (Non-Patent References 1 and 2).

As treatment of such osteoarthritis, Autologous Chondrocyte Implantation (ACI) and Matrix-Induced Autologous Chondrocyte Implantation (MACI) have been practiced, which utilize chondrocytes derived from healthy cartilage tissue collected from a part of articular cartilage that receives no load. Such a transplantation is effective when there is a healthy cartilage tissue around the transplantation site as in a case of a traumatic cartilage damage of knee. However, when such a transplantation is practiced to a degenerated site where the cartilage tissue homeostasis has been destroyed, as in the case of osteoarthritis, although a temporal improvement can be observed, the chondrocytes within the transplant would die soon due to apoptosis, or a formation of fibrous cartilage would occur, providing poor therapeutic effect (Non-Patent Reference 3).

Thus, in treatment of osteoarthritis, there is a requirement for a therapy that is capable of normalizing the degenerated state of cartilage tissue, repairing and maintaining it for a long time period.

### [Prior Art References]

### [Non-Patent References]

[Non-Patent Reference 1] Chen et al.,Bone Res. 2017 Jan 17;5:16044. doi: 10.1038/boneres.2016.44. eCollection 2017.
[Non-Patent Reference 2] Zhang et al., Bone Research volume4, Article number: 15040,2016
[Non-Patent Reference 3] Niemeyera.et al. The Knee, Volume 23, Issue 3, June 2016, Pages 426-435

### [Summary of the Invention]

### [Problems to be Solved by the Invention]

An object of the present invention is to provide a method of producing a chondrocyte culture with high tissue-regenerating ability, a chondrocyte culture produced by said method, and a therapeutic method using the chondrocyte culture.

### [Means to Solve the Problems]

Namely, the present invention relates to those listed below:
[1] A method of producing a chondrocyte culture with high tissue-regenerating ability, the method comprising a step of culturing a cell population separated from cartilage tissue with a thermoreversible gelation polymer.
[2] The method according to [1], wherein the tissue-regenerating ability is an ability of the chondrocyte culture to express one or more genes selected from SOX9, COL2A1, miR140 and miR21, an ability to retain miRNAs, stem cell content, or an ability to secret hyaluronic acid.
[3] The method according to [2], wherein the miRNA is miR140.
[4] The method according to [2] or [3], wherein the stem cell is a pluripotent stem cell or a somatic stem cell with high differentiation potential.
[5] The method according to any one of [2] to [4], wherein the stem cell content is the content of 1-2 fucose or α2-6 sialic acid in the chondrocyte culture.
[6] The method according to any one of [1] to [5], wherein the cartilage tissue is derived from a subject who is at or more than the age of 50 and has osteoarthritis.
[7] The method according to any one of [1] to [6], wherein the thermoreversible gelation polymer is a thermoreversible gelation polymer with no added growth factor other than serum.
[8] A chondrocyte culture produced by the method according to any one of [1] to [7].
[9] A method of treating a disease in a subject, comprising applying an effective amount of the chondrocyte culture according to [8] to a subject in need thereof.
[10] The method of treating according to [9], wherein the disease is osteoarthritis.

[1] A TGP-containing composition for repairing a damaged cartilage tissue.
[2] A composition according to [1], wherein the damaged cartilage tissue is a cartilage tissue damaged by osteoarthritis.
[3] A composition according to [1] or [2], wherein the repairing is normalization of the degenerated state of the damaged cartilage tissue.
[4] A composition according to any one of [1] to [3], wherein the repairing is represented by one or more indices selected from the group consisting of chondrocyte growth, promoting secretion or retaining of hyaluronic acid, promoting secretion of miR140, an increase in CD44-positive cells, and growth of mesenchymal stem cells (MSCs).
[5] A composition according to any one of [1] to [4], wherein the repairing is carried out *in vitro* or *in vivo.*
[6] A composition according to any one of [1] to [5], comprising one or more tissues or cells selected from the group consisting of chondrocyte, chondrocyte progenitor cell or MSCs, a chondrocyte culture and a cartilage tissue.
[7] A composition according to any one of [1] to [6], wherein the chondrocyte or cartilage tissue is an autologous cell or an autologous tissue.
[8] A method of producing a chondrocyte culture and a culture secretion, the method comprising a step of culturing a cell derived from a cartilage tissue of an elderly person in a TGP-containing composition.
[9] The method according to [8], wherein the cartilage tissue is a cartilage tissue damaged by osteoarthritis of knee cartilage.
[10] The method according to [8] or [9], further comprising a step of separating the chondrocyte culture and the culture secretion.
[11] A method of expanding or maintaining an undifferentiated cell, the method comprising a step pf culturing cells which contain a cell obtained from cartilage tissue with a TGP-containing composition.
[12] The method according to [11], wherein the undifferentiated cell is expanded or maintained in a chondrocyte culture.
[13] The method according to [11] or [12], further comprising a step of adding an undifferentiated cell.
[14] The method according to any one of [11] to [13], wherein the undifferentiated cell is an MSC.
[15] The method according to any one of [11] to [14], wherein the cartilage tissue is a cartilage tissue damaged by osteoarthritis.
[16] An undifferentiated cell produced by the method according to any one of [11] to [15].
[17] A TGP-containing composition for preventing degradation of a macromolecular component secreted from a cell.
[18] A method of producing a macromolecular component secreted from a cell, comprising a step of culturing a cell in a TGP-containing composition.
[19] The composition or method according to [17] or [18], wherein the macromolecular component secreted from a cell is hyaluronic acid.
[20] The composition or method according to any one of [17] to [19], wherein the cell is a human cell, and the human cell is one or more cells selected from a group consisting of a human smooth muscle cell, human chondrocyte, human fibroblast, human adipocyte, and human synoviocyte.

### [Effects of the Invention]

The chondrocyte culture produced by the method of the present invention not only has a high engrafting property for its abundant ECM and has function and tissue structure of a heathy cartilage, but it also is capable of normalizing the degenerated state of a cartilage tissue that is characterized in inflammation, ECM degradation, etc. Furthermore, the chondrocyte culture contains a number of stem cells which contribute to cartilage regeneration. Therefore, it exhibits effects of repairing a cartilage tissue in a degenerated state as observed in osteoarthritis and maintaining it for a prolonged period.

### [Brief Description of Drawings]

[Fig.1] Fig.1 shows images of sample tissue obtained by plate culture (Comparative Example 1) and TGP culture (Working Example 1) .
[Fig.2] Fig.2 shows CD44 immunostaining images of samples obtained by plate culture (Comparative Example 1) and TGP culture (Working Example 1).
[Fig.3] Fig.3 shows changes in the amount of α2-6 sialic acid that reacts to SNA being present on the plasma membrane of TGP culture sample.
[Fig.4] Fig.4 shows changes in the amount of α2-6 sialic acid that reacts to SSA being present on the plasma membrane of TGP culture sample.
[Fig.5] Fig.5 shows changes in the amount of α2-6 sialic acid that reacts to TJA-1 being present on the plasma membrane of TGP culture sample.
[Fig.6] Fig.6 shows changes in the amount of α1-3 fucose that reacts to UEA-1 being present on the plasma membrane of TGP culture sample.

### [Mode for Carrying out the Invention]

Hereinbelow, the present invention will be explained in detail.

Unless otherwise defined herein, all technical and scientific terms used herein have the same meanings as being ordinarily understood by a skilled person in the art. All patents, applications and other publications and information are incorporated herein by reference in their entirety. If there is a conflict between the description of a publication referred herein and the present specification, the description of the present specification shall override.

The present invention is a method of producing a chondrocyte culture with high tissue-regenerating ability, the method comprising a step of culturing a cell population separated from cartilage tissue with a thermoreversible gelation polymer. "Cartilage tissue" in the present disclosure refers to a cartilage-derived tissue. Cartilage comprises, without being limited, an articular cartilage, epiphyseal plate, costal cartilage, tracheal cartilage, laryngeal cartilage, sacroiliac joint, temporomandibular joint, sternoclavicular joint, intervertebral disc, pubic symphysis, articular meniscus, articular disc, external auditory canal, eustachian tube, auricular cartilage and epiglottic cartilage, and the like. The cartilage tissue may be derived from any organisms. A human cartilage tissue is preferred because it causes less rejection reaction upon transplanting a bone tissue culture to a damaged human cartilage. It may be an autologous tissue or an allograft tissue, though an autologous tissue is preferred because it causes less rejection reaction upon applying to a subject.

The cartilage tissue to be used in the present invention is capable of forming a chondrocyte culture which has a tissue structure that is similar to a healthy cartilage tissue, regardless of the state of the subject from which the cartilage tissue is originated. Therefore, the cartilage tissue may be derived from a subject who is at any state. For instance, it may be an articular cartilage collected from a subject having osteoarthritis. A site for collecting such articular cartilage may be a part that does or does not receive a load by body weight. The cartilage tissue may be collected from a loaded part that has been damaged by osteoarthritis.

The chondrocyte culture produced by the present invention does not give rise to an aberration such as calcification even after culturing for a long time period. Therefore, for example, it is possible to collect the cartilage tissue from a Grade 1 subject, culture it until the grade advances to Grade 3 or more, then transplant the produced chondrocyte culture to the subject. Accordingly, the OA grade of the subject from whom the cartilage tissue is to be collected may be any one of Grades 1 to 4.

The age of the subject from whom the cartilage tissue is to be collected is not particularly limited. Because the chondrocyte culture produced by the present invention has a high regeneration ability, the cartilage tissue may be derived from a subject who is, for example, 0 to 39 years old, or 40 years old or older, 50 years old or older, 60 years old or older, 70 years old or older, 80 years old or older, or 90 years old or older.

The method for collecting a cartilage tissue may be an ordinary method that is used in the art when a biological sample is collected. The cartilage tissue may be collected from a cartilage using, without being limited, a scalpel, forceps, and a biopsy punch.

In a method of the present invention, the "cell population separated from cartilage tissue" means a cell population that is composed of the same or different types of cells obtained by a separation processing (e.g., an enzyme treatment, a morcellation) for a cartilage tissue. The cell population is not limited as long as it is a cell population contained in a cartilage tissue, including chondrocytes, chondrocyte progenitor cells, mesenchymal stem cells (MSCs) and pluripotent stem cells, and the like.

In the separation processing for a cartilage tissue, a method that is usually used in the art as a separation processing for a biological tissue, for example, an enzymatic method or a physical method, can be used. The enzymatic method uses, although not being limited thereto, one or more enzyme(s) to incubate the cartilage tissue at a temperature at which the cells constituting the cartilage tissue is viable (e.g., 30 °C). The separation processing by a enzymatic method can use an enzyme that is usually used in the art when cells are separated from a cartilage tissue under its corresponding processing condition of temperature, concentration and time.

Enzymes are not limited as long as the cartilage tissue can be separated, and dispase I or II, collagenase I or II, metalloprotease, trypsin, hyaluronidase, pepsin, aminopeptidase, lipase, amylase and the like may be used. In view of being capable of degrading cartilage tissue in a short time with minimum invasion, as the enzyme(s), preferably dispase I or II, collagenase, metalloprotease, accutase or trypsin may be used alone or in combination of two or more.

When trypsin is used, it may be, without being limited, in a form of trypsin-EDTA at 0.05 % to 2.5 %, 0.1 % to 1 %, 0.15 % to 0.3 %. In view of being capable of separating with minimum invasion, it is preferably at 0.2 to 0.25 %. The time of separation processing with trypsin-EDTA may be, without being limited, between 1 minute and 2 hours, between 3 minutes and 1 hour, or between 10 minutes and 40 minutes. In view of avoiding damages to stem cells, it is preferably 30 minutes.

When collagenase II is used, without being limited, it is preferably at 0.1 to 30 mg/ml, 0.5 to 10 mg/ml, 0.75 to 5 mg/ml. In view of being capable of separating with minimum invasion, it is particularly preferably at 0.8 to 2 mg/ml. The time of separation processing is not limited as long as the cells are not killed, and may be, for example, 1 to 24 hours, 3 to 20 hours, 7 to 18 hours, or 10 to 16 hours. In view of separating stem cells with minimum invasion, it is preferably 12 to 16 hours.

When trypsin and collagenase II are used in combination, the concentration and treatment time for each may be combined in any combination without limitation. In view of separating stem cells with minimum invasion while maintaining chondrocyte phenotype, a combination of treating with trypsin-EDTA (0.25 % solution) at 37 °C for 30 minutes and collagenase II solution (1 mg/ml) at 37 °C for 12 to 16 hours is preferred.

Physical methods include, although not being limited thereto, methods of morcellating or fracturing a cartilage tissue using, a scalpel, sonication, homogenizer, strainer, etc. Physical methods may optionally be combined with the enzymatic methods as detailed above.

The cells that were separated by the separation processing and the pieces of the tissue, matrix, etc. that were not fully separated may be fractionated using any known method such as a filter or cell strainer. By fractionation, a cell population that is uniform in size or function is obtained.

In a method of the present invention, the thermoreversible gelation polymer (also referred herein as "TGP") refers to a macromolecule that has a characteristic of being capable of generating a crosslinked or meshwork structure in thermoreversible manner, and, based on this structure, forming a hydrogel that retains a separated liquid body therein in thermoreversible manner. Moreover, the hydrogel refers to a gel comprising the crosslinked or meshwork structure made of the macromolecule and water supported or retained within the structure. In the present invention, the thermoreversible gelation polymer forms an environment that is similar to the cartilage tissue in a living body owing to the crosslinked or meshwork structure that is unique to the thermoreversible gelation polymer. Therefore, any polymer can be used in the method of the present invention, as long as it is a thermoreversible gelation polymer.

### (Sol-Gel Transition Temperature)

In the present invention, definitions and measurement of the "sol state", "gel state", and "sol-gel transition temperature" are based on the definitions and methods described in a literature (H. Yoshioka et al., Journal of Macromolecular Science, A31(1), 113 (19 94)). That is, the dynamic modulus of elasticity of a sample at a observation frequency of 1 Hz is measured while gradually changing the temperature from lower temperature to higher temperature (1 °C/min), and the temperature at the point where the storage modulus of elasticity (G', elastic term) exceeds loss modulus of elasticity (G", viscosity term) is defined as the sol-gel transition temperature. In general, it is defined that sol is a state where G" > G', whereas gel is a state where G" < G'. For measuring this sol-gel transition temperature, the following measurement conditions can be used as suitable.

### <Conditions For Measurement of Dynamic Modulus/Loss Modulus of Elasticity >

Measuring apparatus (trade name): Controlled Stress Rheometer AR500, TA Instruments
Concentration of the sample solution (or separation solution) (as the concentration of "hydrogel-forming macromolecule having sol-gel transition temperature"): 10 (weight) %
Amount of the sample solution: Approximately 0.8 g
Shape/dimensions of the measuring cell: Acrylic parallel disk (diameter = 4.0 cm), gap = 600 µm
Measuring frequency: 1 Hz
Applied stress: Within a linear region

In the present invention, the above-mentioned sol-gel transition temperature is preferably higher than 0 °C and is not higher than 37 °C, more preferably higher than 5 °C and not higher than 35 °C (in particular, higher than 10 °C and not higher than 33 °C). A TGP that has such suitable sol-gel transition temperature can easily be selected from the specific compounds mentioned later according to the screening method described above (Sol-Gel Transition Temperature Measuring Method).

The TGP of the present invention is not particularly limited as long as it exhibits a thermoreversible sol-gel transition as mentioned above (that is, it has a sol-gel transition temperature). As specific examples of the macromolecule which has a sol-gel transition temperature in aqueous solution and indicates sol state at a temperature below the transition temperature in a reversible manner, for example, polyalkylene oxide block copolymers represented by a block copolymer of polypropylene oxide and polyethylene oxide; etherified celluloses such as methyl cellulose and hydroxypropyl cellulose; and chitosan derivatives (K.R.Holme.et al. Macromolecules,24,3828(1991)) are known.

### (Suitable Hydrogel Forming Macromolecules)

A hydrogel forming macromolecule that can suitably be used as the TGP of the present invention which utilizes hydrophobic bonding for forming a crosslinkage preferably consists of a plurality of blocks having clouding points bound by (a) hydrophilic block(s), in view of being capable of avoiding separation of the TGP and the medium and stably maintaining the surrounding of a cell population.

The hydrophilic block is preferably present for a reason that the hydrogel will be water-soluble at a temperature below the sol-gel transition temperature. The plurality of blocks having clouding points are preferably present for a reason that the hydrogel will be turned into a gel state at a temperature above the sol-gel transition temperature.

In other words, because a block having a clouding point dissolves in water at a temperature below the clouding point and turns into water-insoluble at a temperature higher than the clouding point, the block plays a role as a crosslinking point composed of a hydrophobic bond for forming a gel at a temperature higher than the clouding point. That is, the clouding point that is resulted from the hydrophobic bond corresponds to the sol-gel transition temperature of the above-described hydrogel.

Note that the clouding point and the sol-gel transition temperature do not necessarily have to be consistent. This is because the clouding points of the above-described "blocks having clouding points" are generally influenced by the binding of the blocks and the hydrophilic block.

The hydrogel that is used in the present invention utilizes its property that not only its hydrophobic bond becomes stronger as temperature rises, but also its change is reversible to the temperature. In view of that more than one crosslinking points can be formed within one molecule forming a gel with excellent stability, and that a chondrocyte culture can stably be grown against reversible temperature change, it is preferred that the TGP has more than one "blocks having clouding points".

On the other hand, the hydrophilic block in the above-described TGP has a function of changing the TGP to water-soluble at a temperature blow the sol-gel transition temperature, and has a function of preventing the hydrogel from aggregating and precipitating due to an excessive increase in hydrophobic bound strength at a temperature higher than the transition temperature, while forming a state of water-containing gel.

Further, it is desirable that the TGP that is used in the present invention is degradable and absorbable *in vivo,* in view of that the chondrocyte culture of the present invention is to be used for cell therapy. That is, the TGP of the present invention is preferably degraded *in vivo* by hydrolysis reaction or enzymatic reaction to generate low-molecular weight entities that are harmless for the living body, absorbed into and excreted from it.

When the TGP of the present invention is composed of a plurality of blocks having clouding points bound to a hydrophilic block, it is preferred that at least one, preferably both of the blocks having clouding points and the hydrophilic block is/are degradable and absorbable *in vivo.*

### (Plurality of Blocks Having Clouding Points)

A block having clouding point is preferably a macromolecule block which exhibits: [solubility in water] - [temperature coefficient] < 0. More specifically, macromolecules selected from the group consisting of polypropylene oxide, copolymers of propylene oxide and other alkylene oxides, poly N-substituted acrylamide derivatives, poly N-substituted methacrylamide derivatives, copolymers of N-substituted acrylamide derivative and N-substituted methacrylamide derivative, polyvinyl methyl ether, and polyvinyl alcohol partial acetate can preferably be used. In view of being capable of stably culturing the cell population or chondrocyte culture of the present invention, poly N-substituted acrylamide derivative is preferred.

In order that the block having clouding point is to be degradable and absorbable *in vivo,* it is effective that the block having clouding point is a polypeptide composed of a hydrophobic amino acid and a hydrophilic amino acid. Alternatively, a polyester-type biodegradable polymer such as polylactic acid and polyglycolic acid can be utilized as a block having clouding point that is degradable and absorbable *in vivo.*

It is preferred that the clouding point of the macromolecule as described above (the block having clouding point) is higher than 4 °C and not higher than 40 °C, in consideration of that the sol-gel transition temperature of the macromolecule to be used in the present invention (the compound comprising a plurality of blocks having clouding points bound to a hydrophilic block) is to be higher than 0 °C and not higher than 37 °C.

Here, measurement of clouding point can be performed, for example, by cooling an aqueous solution of approximately 1 % (mass) of the macromolecule described above (the block having clouding point) to form a transparent uniform solution, gradually raising the temperature (temperature rising rate at approximately 1 °C/min) thereafter, and defining the point where the solution turns clouded for the first time as the clouding point.

Specific examples of poly N-substituted acrylamide derivatives and poly N-substituted methacrylamide derivatives that can be used in the present invention are listed below: poly-N-acryloyl piperidine; poly-N-n-propyl methacrylamide; poly-N-isopropyl acrylamide; poly-N,N-diethylacrylamide; poly-N-isopropyl methacrylamide; poly-N-cyclopropyl acrylamide; poly-N-acryloyl pyrrolidine; poly-N,N-ethylmethyl acrylamide; poly-N-cyclopropyl methacrylamide; and poly-N-ethyl acrylamide. In view of being capable of stably culturing the cell population or chondrocyte culture of the present invention, poly-N-isopropyl acrylamide is preferred.

The macromolecule described above may be a solo polymer (homopolymer), or may be a copolymer of a monomer and (an)other monomer(s) which constitute(s) the polymer described above. As the other monomer(s) which constitute(s) such a copolymer, either a hydrophilic monomer or hydrophobic monomer can be used. In general, copolymerization with a hydrophilic monomer increases the clouding point of the product, whereas copolymerization with a hydrophobic monomer decreases the clouding point of the product. Therefore, a macromolecule having a desired clouding point (for example, a clouding point that is higher than 4 °C and not higher than 40 °C) can also be obtained by selecting these monomers to be copolymerized.

### (Hydrophilic Monomers)

The hydrophilic monomer described above includes, but is not limited to, N-vinyl pyrrolidone, vinyl pyridine, acrylamide, methacrylamide, N-methyl acrylamide, hydroxyethyl methacrylate, hydroxyethyl acrylate, hydroxymethyl methacrylate, hydroxymethyl acrylate, acrylic acid and methacrylic acid having an acidic group and salts thereof, vinyl sulfonate, styrenesulfonic acid, etc., as well as N,N-dimethylaminoethyl methacrylate, N,N-diethylaminoethyl methacrylate, N,N-dimethylaminopropyl acrylamide having a basic group and salts thereof.

### (Hydrophobic Monomers)

On the other hand, the hydrophobic monomer described above includes, but is not limited to, such as acrylate derivatives and methacrylate derivatives such as ethyl acrylate, methyl methacrylate, glycidyl methacrylate, N-substituted alkyl methacrylamide derivatives such as N-n-buthyl methacrylamide, vinyl chloride, acrylonitrile, styrene and vinyl acetate.

### (Hydrophilic Blocks)

On the other hand, the hydrophilic block to be bound to a block having clouding point described above includes, in specific, such as methyl cellulose, dextran, polyethylene oxide, polyvinyl alcohol, poly N-vinylpyrrolidone, polyvinyl pyridine, polyacrylamide, polymethacrylamide, poly N-methyl acrylamide, polyhydroxymethyl acrylate, polyacrylic acid, polymethacrylic acid, polyvinyl sulfonate, polystyrene sulfonate and salts thereof; poly N,N-dimethylaminoethyl methacrylate, poly N,N-diethylaminoethyl methacrylate, poly N,N-dimethylaminopropyl acrylamide and salts thereof. In one embodiment of the present invention where poly-N-isopropyl acrylamide is used as plurality of blocks having clouding points in the heat-reversible gel, polyethylene oxide is preferred as the hydrophilic block that is bound to the acrylamide, because it forms an environment that is similar to the biological environment within the cartilage tissue, thereby increasing the proliferative capacity of cell population.

It is desired that the hydrophilic block is degraded, metabolized and secreted *in vivo.* Hydrophilic biological macromolecules are preferably used, including proteins such as albumin and gelatin, hyaluronic acid, heparin, chitin, polysaccharides such as chitosan.

The methods of binding the blocks having clouding points and hydrophilic block described above is not particularly limited, though it can be carried out by, for example, introducing a polymerizable functional group (e.g., acryloyl group) into one of the blocks described above, and co-polymerizing a monomer that gives the other of the blocks. Moreover, a binding product of the blocks having clouding points and the hydrophilic block described above can also be obtained by block copolymerization of monomers that give the blocks having clouding points and monomers that give the hydrophilic block. Alternatively, the binding of the blocks having clouding points and the hydrophilic block can also be carried out by introducing in advance a functional group that is reactive to both blocks (e.g., hydroxy group, amino group, carboxyl group, or isocyanate group, etc.) and binding them though a chemical reaction.

In this case, more than one reactive functional groups are usually introduced into the hydrophilic block. Moreover, in a case of the binding of a polypropylene oxide having a clouding point and a hydrophilic block, the propylene oxide and a monomer that constitutes the "other hydrophilic block" (e.g., ethylene oxide) are repeatedly polymerized in sequence by, for example, an anionic polymerization or cationic polymerization, resulting in a block copolymer in which the polypropylene oxide and the "other hydrophilic block" (e.g., polyethylene oxide) are bound to each other.

Such block copolymer can also be obtained by introducing a polymerizable group (e.g., acryloyl group) to a terminal of the polypropylene oxide, then co-polymerizing a monomer that constitutes the hydrophilic block. Furthermore, the macromolecule to be used in the present invention can also be obtained by introducing into the hydrophilic block a functional group that is capable of binding to the functional group (e.g., hydroxy group) on the terminal of the polypropylene oxide and reacting them. Moreover, the TGP to be used in the present invention can also be obtained by coupling a material such as Pluronic^{®} F-127 (Trade name, ADEKA CORPORATION), in which polypropylene glycols are coupled to both terminals of a polypropylene glycol.

The macromolecule of the present invention, in this embodiment in which the macromolecule comprises a block having clouding point, will be completely dissolved in water and exhibit sol-state at a temperature below the clouding point, because the above-described "block having clouding point" that is present within the molecule and the hydrophilic block are both water-soluble. However, if the temperature of this aqueous solution of the macromolecule is heated to a temperature above the clouding point described above, the "block having clouding point" that is present within the molecule will become hydrophobic, and will be associated between other molecules due to hydrophobic interaction.

On the other hand, because the hydrophilic block is still water-soluble at this time (upon being heated the temperature), the macromolecule of the present invention will, in water, form a hydrogel that has a three-dimensional network structure in which the hydrophobic association point between the blocks having clouding points is the crosslinking point. When cooling this hydrogel again to the temperature that is below the clouding points of the "block having clouding point" that is present within the molecule, the block having clouding point will become water-soluble, resulting in the release of the crosslinking point by the hydrophobic association and the loss of hydrogel structure, so that the TGP of the present invention will become a perfect aqueous solution again.

Thus, in preferred embodiments, the sol-gel transition of the macromolecule of the present invention is fully reversible in response to the temperature change, because it is based on the reversible change between hydrophilic and hydrophobic states at the clouding point of the block having clouding point that is present within the molecule. According to the investigation by the present inventors, it is considered that a delicate balance of hydrophilichydrophobic states of the TGP described above in water contributes to the stability of cells when the cells are preserved at low temperature in water.

### (Gel Solubility)

As mentioned above, the hydrogel-forming macromolecule of the present invention comprising at least a macromolecule having a sol-gel transition temperature in aqueous solution exhibits being substantially water-insoluble at a temperature that is higher than the sol-gel transition temperature (d °C), and exhibits being water-soluble in a reversible manner at a temperature that is lower than the sol-gel transition temperature (e °C).

The high temperature (d °C) described above is preferably a temperature that is higher by 1 °C or more, further preferably a temperature higher by 2 °C or more (in particular 5 °C or more) than the sol-gel transition temperature. The above-described "substantially water-insoluble" means that the amount of the above-described macromolecule that is dissolved in 100 ml of water at the above-described temperature (d °C) is preferably 5.0 g or less (furthermore 0.5 g or less, in particular 0.1 g or less).

On the other hand, the above-described low temperature (e °C) is preferably a temperature that is lower than the sol-gel transition temperature (in absolute value) by 1 °C or more, further preferably a temperature that is lower by 2 °C or more (in particular 5 °C or more). The above-described "water-soluble" means that the amount of the above-described macromolecule that is dissolved in 100 ml of water at the above-described temperature (e °C) is preferably 0.5 g or more (furthermore 1.0 g or more). Furthermore, "exhibits being water-soluble in a reversible manner" refers to that the aqueous solution of the TGP described above exhibits being water soluble as described above at a temperature lower than the sol-gel transition temperature even after once being gelled (at a temperature that is higher than the sol-gel transition temperature).

The above-described macromolecule preferably exhibit in its 10 % aqueous solution at 5 °C a viscosity of 10 to 3,000 centipoise (furthermore 50 to 1,000 centipoise). Such viscosity is preferably measured, for example, under the following measuring conditions:
Viscometer: Controlled stress rheometer (model name: AR500, TA Instruments)

| | |
|---|---|
| Rotor diameter: | 60 mm |
| Rotor shape: | Parallel plate |

After the aqueous solution of the TGP of the present invention is gelled at a temperature that is higher than the sol-gel transition temperature, the resulting gel will not substantially be dissolved even being immersed in a large amount of water. The above-described property of the hydrogel formed by the TGP described above can be confirmed, for example, as follows:
Namely, 0.15 g of the TGP is dissolved in 1.35 g of distilled water at a temperature that is lower than the above-described sol-gel transition temperature (e.g., on ice) to generate an aqueous solution of 10 wt%, which is poured into a plastic petri dish having a diameter of 35 mm, warmed to 37 °C, thereby forming a gel of 1.5mm thick in the petri dish. Then the weight of the whole petri dish including the gel (f g) is measured. Next, the whole petri dish including the gel is left still in 250 ml of water at 37 °C for 10 hours, and then the weight of the whole petri dish including the gel (g g) is measured to assess the presence or absence of dissolution of the gel from gel surface. At this point, in the hydrogel-forming macromolecule the present invention, the percentage of weight loss of the above-described gel, i.e., (f-g) /f is preferably 5.0 % or less, further preferably 1.0 % or less (in particular 0.1 % or less).

Once the aqueous solution of the TGP of the present invention is gelled at a temperature that is higher than the above-described sol-gel transition temperature, the resulting gel will not be dissolved for a long time period even being immersed in a large amount (approximately 0.1 to 100 times the gel in volume ratio) of water. Such property of the macromolecule to be used in the present invention is achieved, for example, by the presence of two or more (a plurality of) blocks having clouding points within the macromolecule.

To the contrary, the present inventors have found that, when a similar gel is produced by using the aforementioned Pluronic^{®} F-127 which is composed of polyethylene oxides bound to both terminals of polypropylene oxide, the gel will completely be dissolved in water after a few hours of standing.

In view of suppressing cytotoxicity to a minimum level at the time of un-gelling, it is preferred to use a TGP that is capable of being gelled at a concentration to water, i.e., {(macromolecule)/(macromolecule + water) x100 (%) of 20 % or less (furthermore 15 % or less, in particular 10 % or less).

The molecular weight of the TGP to be used in the present invention is preferably 30,000 or higher and not higher than 30,000,000, more preferably 100,000 or higher and not higher than 10,000,000, further preferably 500,000 or higher and not higher than 5,000,000.

In a method of the present invention, in the step of culturing a cell population with a Thermoreversible Gelation Polymer (TGP), a cell population that has been separated from cartilage tissue can be mixed with the thermoreversible gelation polymer and cultured by a conventional method. In typical, a method can be used in which a cell population that has been separated from cartilage tissue is dispersed in a TGP solution at a temperature that is lower than the sol-gel transition temperature, and the TGP is gelled at a temperature that is higher than the sol-gel transition temperature, before adding a medium such as a culture medium solution and culturing it. By adding a medium to the gelled TGP, it becomes capable of stably supplying nutrients to the cell population in the gel only by regularly changing such medium.

The culturing in the present invention can be carried out under a condition that is usually used in the art. For instance, a typical culturing condition includes culturing at 37 °C in 5% CO₂. Moreover, culturing can be carried out under a normal air pressure (atmospheric pressure). The duration of culturing is not particularly limited because culturing of a cell population for a prolonged period will not result in an abnormal chondrocyte culture such as those with calcification. The duration of culturing can be, for example, 4 days or longer, 8 days or longer, 16 days or longer, 21 days or longer, 32 days or longer, 48 days or longer, 64 days or longer, 72 days or longer, 90 days or longer, 102 days or longer, 114 days or longer, 126 days or longer, 140 days or longer, 160 days or longer, 180 days or longer, 250 days or longer. In view of sufficient ECM formation, it is preferably 21 days or longer, more preferably 32 days or longer, further preferably 72 days or longer.

Culturing can be carried out in a vessel of any size and shape. The medium for dissolving the TGP and the medium to be added to the gelled TGP (also herein referred to as a "liquid medium") are not particularly limited as long as they are capable of maintaining cell viability, though those comprising amino acids, vitamins and electrolytes as principal ingredients can typically be utilized. In the present invention, the medium for dissolving the TGP and the medium to be added to the gelled TGP may be common or different.

In one embodiment of the present invention, the medium for dissolving the TGP and the medium to be added to the gelled TGP are based on a basal medium for cell culturing. Such basal media include without limitation such as, for example, DMEM, MEM, F12, DME, RPMI1640, MCDB (MCDB102, 104, 107, 120, 131, 153, 199, etc.), L15, SkBM, RITC80-7, CnT-PR. Many of these basal media are commercially available and their compositions have also been known. The basal medium may be used in its standard composition (e.g., as it is commercially available), or its composition may appropriately be changed according to the cell species and cell conditions. Accordingly, the basal medium to be used in the present invention is not limited to those of known compositions, but includes those in which one or more ingredient(s) are added, removed, increased or decreased.

The medium may comprise, apart from those described above, one or more additives such as serum, growth factors (e.g., FGF-2, TGF-b1, etc.), steroid agent components, selenium components. The serum may be a xenogeneic serum or an allogeneic serum. An allogeneic serum is preferred, of which an autologous serum is further preferred. Moreover, the medium does not comprise any growth factor within the medium apart from the growth factors contained in the allogeneic serum. When a TGP dissolved in such a medium is used for culturing a cell population, it means that the cell population is to be cultured with a TGP in which no growth factor has been added other than those from the allogeneic serum. The concentration of the serum is not particularly limited, and the medium to be added to the TGP may comprise 3 %, 5 %, 10 % or 20 % of the serum. The concentration is preferably 10 %. In one embodiment of the present invention, the medium for dissolving the TGP and the medium to be added to the gelled TGP may consist of common ingredients.

The "chondrocyte culture" that is produced by a method of the present invention refers to a cell population comprising chondrocytes which has been obtained by culturing a cell population separated from cartilage tissue.

In one embodiment, the chondrocyte culture produced by the method of the present invention may have a tissue structure that resembles a cartilage tissue in which the intercellular space of the cell population is filled with ECM.

The chondrocyte culture obtained by the method of the present invention has a higher tissue-regenerating ability as compared to a chondrocyte culture cultured by a method which does not comprise a step pf culturing the cell population with the thermoreversible gelation polymer (herein referred to as a "control culture").

The tissue-regenerating ability means a tissue-regenerating ability of a cartilage tissue to which the cell population has been applied.

In one embodiment, the tissue-regenerating ability can be quantified, for example, by applying (transplanting) the chondrocyte culture to the cartilage tissue of the subject, and after a predetermined time observing the state of the tissue of the applied site (transplant recipient site), for example, the size of the tissue, microstructure of the tissue, the ratio of damaged tissue to normal tissue, functions of the tissue, etc., and scoring these.

In one embodiment, the tissue-regenerating ability may be an ability of expressing one or more genes selected from SOX9, COL2A1, miR140 and miR21. The chondrocyte culture produced by the method of the present invention expresses one or more genes selected from SOX9, COL2A1, miR140 and miR21 at a higher level than the control culture. Therefore, the present invention may be a method for increasing an ability of a cell population separated from cartilage tissue to express one or more genes selected from SOX9, COL2A1, miR140 and miR21.

SOX9 or COL2A1 has been known as a gene that is expressed at the time of tissue formation of a normal cartilage. Moreover, mir-140-3p or -5p, or miR21-5p, which is derived from miR140 or miR21, is effective for normalizing the degenerated state of cartilage tissue which is characterized in an inflammation within cartilage tissue, ECM degradation, etc. caused in osteoarthritis (see, such as Karlsen et al., Mol Ther Nucleic Acids. 2016 Oct 11;5(10), Miyaki et al., Arthritis Rheum. 2009 Sep;60(9):2723-30, Si et al., Osteoarthritis Cartilage. 2017 Oct;25(10):1698-1707, Miyaki et al., Genes Dev. 2010 Jun 1;24(11):1173-85, Hai et al,. J Orthop Surg Res. 2019; 14: 118).

Specifically, a high expression of miR140 in cartilage tissue increases mir-140-3p or 5p in the cartilage tissue, which results in the normalization of the degenerated state of the cartilage tissue via, without being limited, decreasing the expression or secretion of ECM catabolic enzymes such as MMP-13 and ADAMTS-5, decreasing the expression or secretion of inflammatory mediators such as IL1B, IL6 and IL8, and increasing the expression of proteins, etc. involved in ECM synthesis such as SOX9, ACAN and chondroitin sulfate N-acetylgalactosaminyltransferase 1 (CSGALNACT1). Therefore, applying the chondrocyte culture of the present invention to cartilage tissue of a subject, particularly a subject having osteoarthritis can not only complement normal cartilage tissue to the applied site, but can also normalize the degenerated state of the cartilage tissue surrounding the applied site, thereby being able to maintain the therapeutic effect for a prolonged period.

Moreover, a high expression of miR21 in cartilage tissue increases mi21-5p in the cartilage tissue, which results in the normalization of the degenerated state of the cartilage tissue via, without being limited, decreasing the expression or secretion of ECM catabolic enzymes such as MMP-13 and ADAMTS-5, and increasing the expression of proteins, etc. involved in ECM synthesis such as COL2A1. Therefore, as with miR140, it can maintain the therapeutic effect for a prolonged period.

Here, a high expression of a gene means that the expression level of a given gene is higher as compared to the control culture by, without being limited, 101 % or more, 105 % or more, 110 % or more, 130 % or more, 140 % or more, 150 % or more, 160 % or more, 170 % or more, 180 % or more, 190 % or more, or 200 % or more.

Procedures for measuring the expression level of a gene are well known in the art. Procedures for measuring the expression level of a protein include, without being limited, for example, Western blotting, EIA, ELISA, RIA, immunohistochemistry, immunocytochemistry using monoclonal or polyclonal antibodies detailed above, flow cytometry, mass spectrometry, etc., and procedures for measuring the expression level of a gene include, without being limited, for example, Northern blotting, Southern blotting, DNA microarray analysis, RNase protection assay, PCRs such as RTPCR and real-time PCR (qPCR), and *in situ* hybridization, respectively.

In the present disclosure, SOX9 (also referred to as CMD1, CMPD1, SRA1, SRXX2 or SRXY10) is a gene coding for SRY-box transcription factor 9, and the gene sequence for human SOX9 has been registered under Accession No.: NM_000346, etc., and the sequence is as indicated by SEQ ID NO.1.

In the present disclosure, COL2A1 (also referred to as ANFH, AOM, COL11A3, SEDC or STL1) is a gene coding for collagen type II alpha 1 chain, and the gene sequence for human COL2A1 has been registered under Accession No.: NM_001844.5, etc., and the sequence is as indicated by SEQ ID NO.2.

In the present specification, a "microRNA (miRNA)" is used to mean an RNA of 10 to 25 bases, which is transcribed as an RNA precursor of a hairpin-like structure, cleaved by a dsRNA cleavage enzyme having RNaseIII-cleaving activity, taken up by a protein complex called RISC and is involved in suppression of mRNA translation. The "miRNA" also encompasses a "miRNA" and a precursor of "miRNA" (pre-miRNA, pri-miRNA), as well as other miRNAs having equivalent biological functions to those of the miRNA encoded thereby, for example, "miRNAs" coding for homologs, mutants such as genetic polymorphisms, and derivatives. The "miRNAs" coding for such precursors, homologs, mutants and derivatives can be identified by miRBase (http://www.mirbase.org/), and can include a "miRNA" having a nucleotide sequence that hybridizes to a complementary sequence for a certain nucleotide sequence under a stringent condition.

In the present disclosure, human mir-140 has been registered under miRbase ID: Stem-loop sequence hsa-mir-140, miRbase Accession No.: MI0000456, and is as indicated by SEQ ID NO.3.

In the present disclosure, human mir-140-3p has been registered under miRbase ID: Mature sequence hsa-miR-140-3p, miRbase Accession No.: MIMAT0004597, and is as indicated by the RNA sequence "uaccacaggguagaaccacgg" indicated by SEQ ID NO.4

In the present disclosure, human mir-140-5p has been registered under miRbase ID: Mature sequence hsa-miR-140-5p, miRbase Accession No.: MIMAT0000431, and is as indicated by the RNA sequence "cagugguuuuacccuaugguag" indicated by SEQ ID NO.5.

In the present disclosure, human mir-21 has been registered under miRbase ID:Stem-loop sequence hsa-mir-21, miRbase Accession No.: MI0000077, and is as indicated by SEQ ID NO.6.

In the present disclosure, human miR21-3p has been registered under miRbase ID: Mature sequence hsa-miR-21-3p, miRbase Accession No.: MIMAT0004494, and is as indicated by RNA sequence "caacaccagucgaugggcugu" indicated by SEQ ID NO.7.

In the present disclosure, human miR21-5p has been registered under miRbase ID: Mature sequence hsa-miR-21-5p, miRbase Accession No.: MIMAT0000076, and is as indicated by RNA sequence "uagcuuaucagacugauguuga" indicated by SEQ ID NO.8.

In one embodiment, the tissue-regenerating ability is an ability of a chondrocyte culture to retain miRNAs. The ability to retain miRNAs is an ability of a chondrocyte culture to retain miRNAs within the chondrocyte culture. The ability to retain miRNAs within the chondrocyte culture may be an ability not to secrete miRNAs upon being cultured, but to retain them within the chondrocyte culture. Because the chondrocyte culture retains miRNAs within it during culture, when the obtained chondrocyte culture is applied to a subject, it can secret miRNAs at a high concentration at and around the applied site. By retaining miRNAs in the chondrocyte culture during cultivation, it is possible to obtain a chondrocyte culture that can secrete miRNAs at a high concentration at and around the applied site when the chondrocyte culture is applied to a subject.

The miRNAs include, without being limited, miRNAs that can normalize the degenerated state of cartilage tissue, such as miR140, miR21, miR-125b,Has-miR-15a, miR-30a, miR-199a, miR-210, miR-221-3p, miR-92a-3p, miR-142-3p, miR-27a, miR-27b, miR26a-5p, miR-26a, miR-26b, miR-373, miR-127-5p, miR-320, miR-9, miR-634, miR-221-3p, miR-370, miR-145, miR-130A, miR-145 and miR-562-5p (see, Zhang.et al.J Arthritis. 2017 Apr;6(2). pii: 239. doi: 10.4172/2167-7921.1000239), and is preferably miR140 (including mir-140-3p and -5p) and miR21 (including miR21-3p and -5p), particularly preferably miR140 (including mir-140-3p and -5p).

The ability of retaining miRNA being high refers to that the miRNA is present in the chondrocyte culture at the time of being cultured, at a concentration that is, without being limited, 101 % or higher, 105 % or higher, 110 % or higher, 130 % or higher, 140 % or higher, 150 % or higher, 160 % or higher, 170 % or higher, 180 % or higher, 190 % or higher, or 200 % or higher as compared to a control culture at the time of being cultured. In one embodiment, the ability of retaining miRNA being high refers to that the miRNA is present in the liquid medium of the chondrocyte culture at the time of being cultured, at a concentration that is, without being limited, 99 % or lower, 95 % or lower, 97 % or lower, 95 % or lower, 90 % or lower, 80 % or lower, 70 % or lower, 60 % or lower, 50 % or lower, 40 % or lower, 30 % or lower, 20 % or lower, 10 % or lower as compared to the medium (liquid medium) of the control culture at the time of being cultured. In one embodiment, the ability of retaining miRNA being high refers to that the miRNA is present in the chondrocyte culture at the time of being cultured at a high concentration as compared to the control culture at the time of being cultured, and that the miRNA is present in the medium of the chondrocyte culture at the time of being cultured, at a low concentration as compared to the liquid medium of the control culture.

In one embodiment, the tissue-regenerating ability may be a content of stem cells in the chondrocyte culture. The chondrocyte culture produced by the method of the present invention contains a higher content of stem cells than the control culture. Therefore, the present invention may be a method for maintaining or expanding stem cells in a cell population separated from cartilage tissue. In one embodiment, the content of stem cells may be, without being limited, an amount or percentage of stem cells occupying in a cell culture. The stem cell is not limited as long as it is a cell having an ability to differentiate into a chondrocyte, and includes such as a chondrocyte progenitor cell, a somatic stem cell such as a mesenchymal stem cell, or a pluripotent stem cell such as an ES cell, iPS cell and ntES cell.

The content of a stem cell can be measured by any method for measuring stem cell known in the art. It can be quantified by, without being limited, by measuring an amount of α1-2 fucose that reacts with a lectin being present on plasma membrane of a cell contained in the chondrocyte culture, such as UEA-1, or an amount of α2-6 sialic acid that reacts with a lectin such as SNA, SSA or TJA-I, or by measuring expression of other cell surface markers or genes that correlate to a change in the amount of the above-described α1-2 fucose acid or the above-described α2-6 sialic acid. A higher level of the above-described α1-2 fucose being present on plasma membrane of the cell contained in the chondrocyte culture means that more pluripotent stem cells are present; a higher level of the above-described α2-6 sialic acid means that more somatic stem cells with high differentiation potential are present. (See, Wang et al, Cell Res. 2011 Nov;21(11):1551-63, Tateno et al, Glycobiology. 2016 Dec;26(12):1328-1337,WO2016006712A1).

The "differentiation potential" refers to that a cell has an ability to change into another cell species such as a precursor cell or a somatic cell under an appropriate differentiation-inducing condition. In general, when a somatic stem cell is cultured for a prolonged period, its differentiation potential tends to be decreased along with its proliferative capacity. The somatic stem cell with decreased differentiation potential cannot change into another cell species such as a precursor cell or a somatic cell regardless of the possession of somatic stem cell markers on its cell surface. Therefore, such somatic stem cell does not have the tissue-regenerating ability.

The chondrocyte culture produced by the method of the present invention has a high content of somatic stem cells with high differentiation potential. The content of somatic stem cells with high differentiation potential may be, without being limited, an amount or percentage of somatic stem cells having high differentiation potential that occupies an unit amount of a cell culture. In certain embodiment, the present invention may be a method of maintaining or increasing the differentiation potential of a somatic stem cell in a cell population separated from cartilage tissue. In another embodiment, the present invention may be a method of maintaining or expanding a somatic stem cell with high differentiation potential in a cell population separated from cartilage tissue. The somatic stem cell is not limited as long as it is a somatic stem cell that is capable of differentiating into a chondrocyte, and may be a chondrocyte progenitor cell, mesenchymal stem cell, etc.

By applying a chondrocyte culture comprising such stem cells or somatic stem cells with high differentiation potential in a large amount to the cartilage tissue of the subject, the applied site can permanently be supplied with chondrocytes differentiated from cells with high differentiation potential, and can thereby repair and maintain normal cartilage tissue for a prolonged period. Moreover, when the stem cell or somatic stem cell with high differentiation potential is a mesenchymal stem cell, it has high abilities of normalizing a damaged cartilage tissue and repairing against damages. Therefore, it can maintain a therapeutic effect, e.g., for protecting chondrocyte against apoptosis, for anti-inflammation for cartilage tissue, for anti-fibrillization, for a prolonged period.

The amount of α1-2 fucose that reacts to a lectin such as UEA-1 present on plasma membrane of a cell contained in the chondrocyte culture, or of α2-6 sialic acid that reacts to a lectin such as SNA, SSA or TJA-I can be measured by, without being limited, using flow cytometry, Western blotting, immunostaining, or Antibody-overlay lectin microarray, etc., using antibodies that specifically react to the above-described lectins or to α1-2 fucose or α2-6 sialic acid that reacts to the above-described lectins. Conventional methods in the art for measuring expression of cell-surface markers or genes can be used as methods of measuring expression of other cell-surface markers or genes which correlate to the change in the amount of the above-described α1-2 fucose acid or α2-6 sialic acid.

The chondrocyte culture produced by the method of the present invention comprises more stem cells or somatic stem cells having high differentiation potential than the control culture. Comprising more stem cells or somatic stem cells having high differentiation potential means that the content of the cells having α1-2 fucose acid or α2-6 sialic acid, or the expression of cell-surface markers or genes that correlate thereto is higher than the control culture by, without being limited, 101 % or more, 105 % or more, 110 % or more, 130 % or more, 140 % or more, 150 % or more, 160 % or more, 170 % or more, 180 % or more, 190 % or more, or 200 % or more.

In one embodiment, the tissue-regenerating ability may be an ability of the chondrocyte culture to secret hyaluronic acid. The chondrocyte culture produced by the method of the present invention has a higher ability to secret hyaluronic acid than the control culture. The present invention therefore may be a method of increasing the secreting ability of a cell population separated from cartilage tissue. Hyaluronic acid has been known to suppressively act to the expression of ECM degrading enzymes, alleviating the release of matrices or anti-inflammatory factors from cartilage, controlling the expression of inflammation-related factors (Ohtsuki.et al.J Orthop Res. 2018 Aug 17. doi: 10.1002/jor.24126) .

Accordingly, applying the chondrocyte culture produced by the method of the present invention to cartilage tissue of the subject can not only complement normal cartilage tissue to the applied site, but can also suppress ECM degradation or inflammation around the applied site, and can thereby normalize the degenerated state of the cartilage tissue around the applied site, repair and maintain normal cartilage tissue for a prolonged period.

The ability to secret hyaluronic acid can be measured by quantifying hyaluronic acid in the medium (liquid medium) or TGP over time. Hyaluronic acid can be quantified by, without being limited, mass spectrometry, liquid chromatography and MALDI-TOF, as well as other commercially available kits.

The ability to secret hyaluronic acid being high means that the ability to secret hyaluronic acid is higher than the control culture by, without being limited, 101 % or more, 105 % or more, 110 % or more, 130 % or more, 140 % or more, 150 % or more, 160 % or more, 170 % or more, 180 % or more, 190 % or more, or 200 % or more.

Furthermore, the chondrocyte culture produced by the method of the present invention is easily engrafted to a damaged site because it contains abundant ECM. Therefore, the chondrocyte culture produced by the method of the present invention can be used for transplantation. Moreover, because it contains miR140 and miR21 at high expression levels, comprises a large number of stem cells, and secretes hyaluronic acid at a high level, it is favorably used for a therapy for Grade II or a higher grade osteoarthritis or for osteoarthritis in an elderly person.

The present invention further encompasses a chondrocyte culture produced by a method of the present invention. The chondrocyte culture of the present invention is as mentioned above.

The present invention also encompasses a method of treating a disease in a subject, wherein the method comprising applying an effective amount of the chondrocyte culture of the present invention to a subject in need thereof. The disease is not limited as long as it is a cartilage-associated disease, and includes osteoarthritis, rheumatoid arthritis, osteosarcoma, femoral head osteonecrosis, acetabular dysplasia, tibial meniscus injury, traumatic arthritis, and a physical cartilage loss or damage due to athletics or an accident.

Methods of applying (administrating) to a subject may be a transplant of the chondrocyte culture. The transplant of the chondrocyte culture can be carried out, without being limited, as follows: the chondrocyte culture is delivered to an affected site by injection to the cartilage tissue or by arthroscopic surgery and incision, etc., and it can be fixed to the affected site by using any tissue adhesive such as fibrin gel or by anastomosis.

In a therapeutic method of the present invention, various additional ingredients, for example, a pharmaceutically acceptable carrier, an ingredient for increasing engrafting rate, etc., and further effective ingredients can be included and applied. Any known ingredients can be used as such additional ingredients, and a person skilled in the art is familiar with these additional ingredients.

Hereinbelow, specific embodiments of the present invention are listed for illustrating the present invention in further detail, though the present invention is not to be limited to these specific examples.

### [Examples]

### [Production Example 1]

N-isopropyl acrylamide 42.0 g and 4.0 g of n-butyl methacrylate were dissolved in 592 g of ethanol. To this, an aqueous solution of 11.5 g of polyethylene glycol dimethacrylate (PD E6000, NOF CORPORATION) dissolved in 65.1 g of water was added, which was warmed to 70°C under a nitrogen airflow. While maintaining 70 °C under a nitrogen airflow, N,N,N',N'-tetramethyl ethylene diamine (TEMED) 0.4 mL and 4 mL of 10% aqueous solution of ammonium persulfate (APS) were added, stirred for 30 minutes to allow for reacting. Further 0.4 mL of TEMED and 4 mL of 10% aqueous solution of APS were added four times at 30-minute intervals to complete the polymerization reaction. The reaction solution was cooled to 5 °C or below, then diluted by adding 5 L of cold distilled water at 5 °C, and concentrated to 2 L using a ultrafiltration membrane of fractionation molecular weight of 100,000 at 5 °C.

The concentrated solution was diluted by adding 4 L of cold distilled water, and the above-described ultrafiltration procedure was performed again. The above-described dilution and ultrafiltration procedures were repeated five more times to remove substances with molecular weight of less than 100,000. Substances which were not filtrated off by these ultrafiltration (residual substances in the ultrafiltration membrane) were collected and lyophilized to give 40 g of TGP of the present invention with molecular weight of more than 100,000. One gram of the TGP of the present invention obtained as above was dissolved in 9 g of distilled water on ice to give a 10 wt% aqueous solution. The storage modulus of elasticity of this aqueous solution was 43Pa at 10 °C, 680Pa at 25 °C, and 1310Pa at 37 °C, as measured by using a control stress rheometer (model name: AR500, TA Instruments) with applied frequency at 1 Hz. This temperature-dependent change in the storage modulus of elasticity was observed repeatedly in a reversible manner.

### 1. Culturing a Cell Population Separated from Cartilage Tissue

### <Sample Collection>

For six patients with Grade III or IV osteoarthritis who had experienced joint replacement of the knee, a part of cartilage tissue (approximately 10 x 5 mm, 3 mm thick) from the resected damaged site was sampled, immersed in PBS containing antibiotics (gentamicin (50 µg/ml), amphotericin (0.25 µg/ml), penicillin (100 Units/ml)/streptomycin (100 µg/ml)) for 30 minutes. The age and sex of each patient are indicated below.

**[Table 1]**

| Sample No. | Age | Sex |
|---|---|---|
| 1059 | 68 | Male |
| 1064 | 79 | Female |
| 1066 | 80 | Female |
| 1067 | 66 | Female |
| 1068 | 84 | Female |
| 1069 | 73 | Female |

### <Separating Cartilage Tissue>

Each tissue piece was cut into 1 mm² or smaller pieces with a scalpel. These small pieces were treated with Tripsin-EDTA solution (0.25%) at 37 °C for 30 minutes, then enzymatically treated with collagenase II solution (1mg/ml) at 37 °C for 12 to 16 hours. It was washed with DMEM solution, then filtered (100µm) and centrifuged (1800 rpm, 10 min). The cell population thus obtained was counted with a hemocytometer. The number of cells were between 1 x 10⁴ and 4 x 10⁴ cells/ml.

### [Comparative Example 1]

To a medium containing 10% autologous serum-containing DMEM solution with antibiotics (gentamicin (50 µg/ml), amphotericin (0.25 µg/ml), penicillin (100 Units/ml)/streptomycin (100 µg/ml)), the cells derived from the cartilage tissue of each sample were dispensed, and cultured at 37 °C in 5 % carbon dioxide incubator. The culture medium was replaced every week, and culture was carried out for 4 to 7 weeks.

### [Working Example 1]

### <Culturing with TGP Gel>

One gram of TGP generated in Production Example 1 was dissolved at 4°C in 9 mL of DMEM to generate 10 % TGP solution, then the cells derived from the cartilage tissue were dispersed therein, which was dispensed into a 6-well plate. After being left still at room temperature to allow for gelling, 7 to 8 ml of a medium containing 10 % autologous serum-containing DMEM solution with antibiotics (gentamicin (50 µg/ml), amphotericin (0.25 µg/ml) , penicillin (100 Units/ml)/streptomycin (100 µg/ml)) and L-ascorbic acid (5 mg/ml) was added, and this was cultured in 5 % carbon dioxide incubator (ESPEC BNA-111). The culture medium was replaced every week, and culture was carried out for 20 weeks.

### <Observation of Cultured Cartilage Tissue>

### (1) Phase-Contrast Microscopy Observation

In Comparative Example 1, normal chondrocytes were confirmed at the beginning of starting culture, though elongated fibroblastlike cells gradually emerged over the course of culturing, which started degenerating or dying from about Day 12 till about Day 20, and were completely degenerated or dead about 4 weeks after starting culturing. For instance, cells were dead at Day 28 in Sample 1059, at Day 23 in Sample 1066, and at Day 18 in Sample 1068.

In Working Example 1, chondrocyte cultures of the size of 200 to 300 µm were confirmed on Day 3 to Day 5 after starting culturing, which grew to the size of 0.5 to 1.0 mm in diameter after 21 days, indicating that the growth was maintained in all samples during 20-week culturing period. No growth of fibroblastlike cells was confirmed in Working Example 1.

### (2) HE Staining

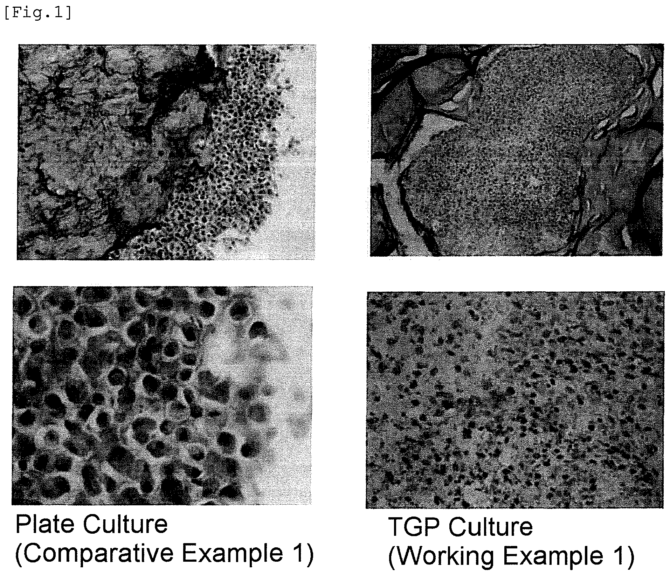

Part of Sample (1059) from Comparative Example 1 was collected on Day 25 after starting culturing, and a part of Sample (1059) of the TGP gel culture from Working Example 1 was collected on Day 49 after starting culturing, fixed with formaldehyde, embedded in paraffin to make tissue blocks, which was subsequently cut into 10 µm to give tissue sections. Their images observed with an optical microscope (x 200) are shown in Fig.1.

No ECM was confirmed in the sample from Comparative Example 1. On the other hand, in the sample from Working Example 1, it was found that chondrocytes were surrounded by ECM, having a tissue structure that is similar to the tissue of a healthy hyaline cartilage.

### (3) CD44 Immunostaining

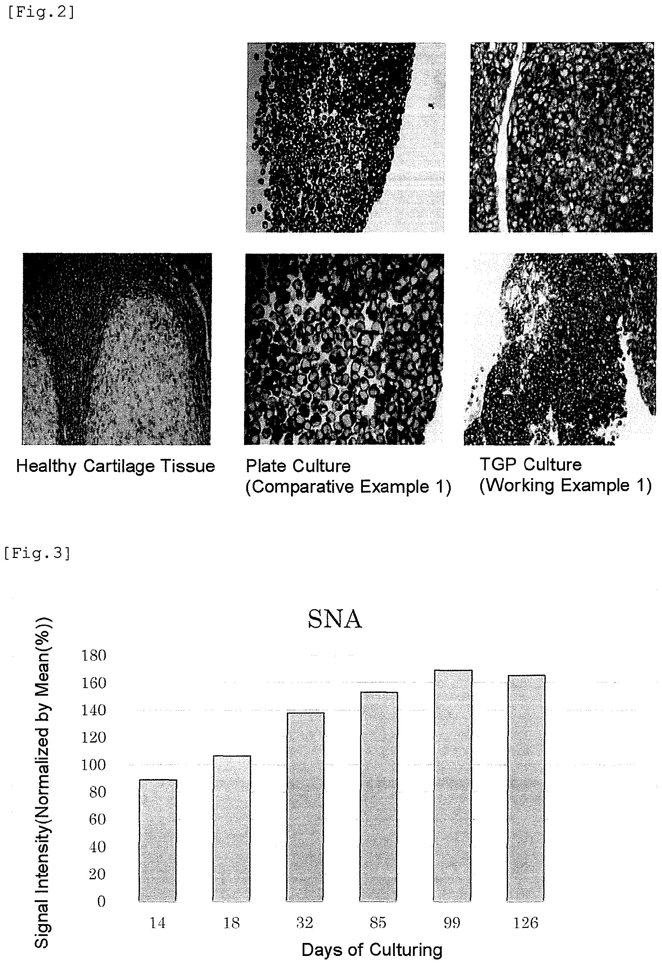

Tissue sections obtained from a healthy cartilage tissue, the samples from Comparative Example 1 and Working Example 1 were fixed with formaldehyde, embedded in paraffin to make tissue blocks, which was subsequently cut into 5 µm to give tissue sections. After deparaffinization, the sections were reacted with a primary antibody, rabbit monoclonal anti-CD44 antibody SP37 (Ventana Medical Systems Inc. USA) at room temperature for 32 minutes, then colored with iView DAB detection kit (Ventana Medical Systems Inc. USA). Results are shown in Fig. 2.

In the chondrocyte culture, CD44-positive chondrocytes and ECM were diffusively present. In the plate culture sample from Comparative Example 1, there were no ECM, but only CD44-positive cells were present.

### (4) Measuring Gene Expression in Each Sample

A part of each of Samples 1059, 1066 and 1068 from Comparative Example 1 was collected on Days 14, 17 and 26 after starting culturing. A part of Samples 1059, 1066 and 1068 was also collected on Day 42 after starting culturing. Note that the collecting date is different in the samples from Comparative Example 1 and Working Example 1. This is because chondrocytes were completely dead on Day 42 in any of the samples from Comparative Example 1, and the used samples were those which could be collected at the last timing during the period in which chondrocyte were viable in these samples.

The collected samples were stored in RNAlater^{®} Stabilization Solution (Invitrogen, CatNo.AM7020) at -20 °C. For a part of these samples, small RNAs (RNAs of 200 bps or smaller) were purified using NucleoSpin^{®} miRNA kit (TaKaRa, U0971), qPCR was performed for U6, miR-21-3p, miR-21-5p, miR-140-3p and miR-140-5p using primers of SEQ ID NOs.9 to 12, mRQ 3' primer, U6 Forward Primer and U6 Reverse Primer (Mir^{-XTM} miRNA qRT-PCR TB Green Kit (TaKaRa, Z8314N) on Thermal Cycler Dice^{®} Real Time System Lite (TP700, TaKaRa). The quantitated value for each miRNA was normalized assuming that the U6 expression level is 1.

Total RNA was purified using miRNAeasy Mini Kit (QIAGEN, 217004) in a similar way. Complementary DNAs were synthesized by reverse transcription using Superscript III reverse transcriptase (Invitrogen, 18080044), and qPCR was performed for GAPDH, SOX9 and COL2A1 using primers of SEQ ID NOs.13 to 16 and TB Green Premix Ex Taq II (Takara,Cat No. RR820S/A/B) on Thermal Cycler Dice^{®} Real Time System Lite. The quantitation value for each gene was normalized assuming that the GAPDH expression level is 1. Primers used are shown in Table 2, the results are shown in Tables 3 to 5.

**[Table 2]**

| Primer | Sequence (5'→3') | SEQ ID NO. |
|---|---|---|
| miR21-3p Fwd | 5' - CAACACCAGTCGATGGCTGT- 3' | 9 |
| miR21-5p Fwd | 5 ' - TAGCTTATCAGACTGATGTTGA - 3' | 10 |
| miR 140-3p Fwd | 5 ' - CAGTGGTTTTACCCTATGGTAG - 3' | 11 |
| miR140-5p Fwd | 5' - TACCACAGGGTAGAACCACGG - 3' | 12 |
| Sox9 Fwd | 5' - GGAGATGAAATCTGTTCTGGGAATG - 3' | 13 |
| Sox9 Rev | 5' - TTGAAGGTTAACTGCTGGTGTTCTG - 3' | 14 |
| Col2a1 Fwd | 5' - CCAGTTGGGAGTAATGCAAGGA - 3' | 15 |
| Col2a1 Rev | 5' - ACACCAGGTTCACCAGGTTCA - 3' | 16 |

**[Table 3]**

| Sample : 1059 | 2D-Day26 | 3D TGP-Day42 | fold |
|---|---|---|---|
| U6(Control) | 1 | 1 | 1 |
| miR21-5p | 1.0968 | 1.909 | 1.74 |
| miR21-3p | 0.0273 | 0.0215 | 0.79 |
| miR140-5p | 0.0131 | 0.0656 | 5 |
| miR140-3p | 0.0281 | 0.1288 | 4.57 |
| GAPDH | 1 | 1 | 1 |
| SOX9 | 0.0041 | 0.1182 | 28.83 |

**[Table 4]**

| Sample :1066 | 2D- Day17 | 3D TGP-Day42 | fold |
|---|---|---|---|
| U6 | 1 | 1 | 1 |
| miRNA21-5p | 0.0728 | 0.3763 | 5.17 |
| miRNA21-3p | 0.0088 | 0.0134 | 1.51 |
| miRNA140-5p | 0.0007 | 0.0248 | 31.34 |
| miRNA140-3p | 0.0024 | 0.0936 | 38.68 |

**[Table 5]**

| Sample :1068 | 2D-Day14 | 3D TGP-Day42 | fold |
|---|---|---|---|
| U6 | 1 | 1 | 1 |
| miR21-5p | 0.816 | 1.4983 | 1.84 |
| miR21-3p | 0.037 | 0.0153 | 0.41 |
| miR140-5p | 0.0073 | 0.0471 | 6.39 |
| miRNA140-3p | 0.0024 | 0.0936 | 38.68 |
| GAPDH | 1 | 1 | 1 |
| SOX9 | 0.0042 | 0.0791 | 18.81 |
| COL2a1 | 0.0000185 | 0.0000654 | 3.54 |

It was found that, in any of Samples 1059, 1066 and 1068, the expression levels of miR-21 and miR-140 were increased and miR21-5p, miR-140-3p and miR-140-5p were greatly increased in samples from Working Example 1 cultured in TGP gel as compared to the samples from Comparative Example 1.

It was also found that, in either of Samples 1059 and 1068 in which SOX9 expression level was measured, SOX9 expression level was greatly increased in the samples from Working Example 1 as compared to the samples from Comparative Example 1. Moreover, it was found that, in 1068 in which COL2A1 was measured, the expression level was increased in the sample from Working Example 1 as compared to that from Comparative Example 1. The expression of miR21, miR-140, SOX9 and COL2A1 have all been known to be increased at the time of healthy cartilage tissue formation. Accordingly, it was found that the chondrocyte culture produced in Working Example 1 is a cartilage tissue having healthy functions. Among those, miR-140 and miR21 have been known to improve the microenvironment in osteoarthritis. Therefore, the chondrocyte culture produced in Working Example 1 can not only stimulate cartilage tissue regeneration at the transplanted part, but can also improve the degenerated state around the transplanted part.

### (5) Quantifying miR140 in Liquid Medium

The liquid media from Samples 1067 and 1068 cultured in Comparative Example 1 and Working Example 1 were collected in 2.0 m on Day 7 and Day 8 after starting culturing, stored in RNAlater^{™} Stabilization Solution (Invitrogen, CatNo.AM7020) at -20 °C. From parts of these samples, small RNAs (RNAs of 200 bps or smaller) were purified using NucleoSpin^{®} miRNA kit (TaKaRa, U0971), and qPCR was performed for miR-140-3p using the primer of SEQ ID NO.3 and mRQ 3' Primer (Mir-X^{™} miRNA qRT-PCR TB Green^{®} Kit (TaKaRa, Z8314N)) on Thermal Cycler Dice^{®} Real Time System Lite (TP700, TaKaRa). The results are shown in Table 6. The mean Ct value is the number of cycles until reaching a predetermined threshold, indicating that, the lower the mean Ct value is, the more miR-140-3p being present in the liquid medium. In the table, "SNP" indicates those which were collected, centrifuged and filtrated before storing, whereas "SNF" indicates those which were stored without such treatments.

**[Table 6]**

| Sample | 2D | | 3D-TGP | |
|---|---|---|---|---|
| | Days of culturing | Mean Ct value | Days of culturing | Mean Ct value |
| 1067- SNP | 7 | 35.95 | 8 | 45.85 |
| 1067-SNF | 7 | 33.96 | 8 | 45.11 |
| 1068-SNP | 7 | 34.76 | 7 | 35.8 |
| 1068 - SNF | 7 | 33.06 | 7 | 37.04 |

From Table 6, it was found that, in either sample, more miR-140-3p is present in the liquid medium of Comparative Example 1 as compared to that of Working Example 1. In view of that the express level of miR-140-3p was higher in the chondrocyte culture of Working Example 1 as compared to Comparative Example 1 in gene expression of (4), it was found that the majority of miR-140-3p that had been expressed in the chondrocyte culture of Working Example 1 was not secreted into the liquid medium, but was retained in the chondrocyte culture. Accordingly, it became clear that a tissue culture with high miRNA content can be obtained by culturing the tissue in the TGP gel.

### (6) Measuring Hyaluronic Acid

In the TGP gel culturing of Working Example 1, 2.0 ml each of the liquid medium was collected on Day 0, Day 32 and Day 113 after starting culturing, each of which was frozen at -20 °C. Hyaluronic acid present in each sample was measured by hyaluronic acid measuring kit (PG Research HA-Kit; Lot. 18H484). The results are shown in Table 7.

Hyaluronic acid, which was not present at the beginning of culturing, was present on Day 32 at 822.8 ng/ml, and on Day 113 at 670.1 ng/ml. From this, it was found that hyaluronic acid was stably secreted from the chondrocyte culture. Therefore, it is confirmed that the chondrocyte culture cultured in the TGP gel is a cartilage tissue indicating a healthy condition.

**[Table 7]**

| Days of culturing | Hyaluronic acid concentration (ng/mL) |
|---|---|
| 32 | 822.8 |
| 113 | 670. 1 |

### (7) Glycan Analysis of Cell Surface of the Cells That Constitute the Chondrocyte Culture

The chondrocyte culture that had been cultured in Working Example 1 was collected during a period of 14 to 126 days after starting culturing, and the TGP gel from each was cooled to 4 °C and collected in the state of an aqueous solution. The sample was dispersed into cells with Tripsin-EDTA (0.25%) solution, and the cells were collected. Next, this was washed by centrifuging with DMEM at 4 °C for 3 times (1800 rpm, 15 min), and the sample for each was frozen for storage. The analysis of the sample was consigned to Glycan Profiling Analysis Service by GlycoTechnica Ltd. In specific, plasma membrane protein was extracted from the dissolved sample, and its concentration was measured by BCA method (TaKaRa BCA Protein Assay Kit). Based on the measured protein concentration, PBSTx was added to dilute the protein such that the concentration becomes 10 µg/mL. To a tube containing 100 µg of Cy3 Mono-reactive Dye Pack (GE Healthcare, Catalog No.: PA23011), 100 µL of the sample (concentration = 10 µg/mL) was added, mixed by pipetting and spun down. The tube was put into a light-shielding bag, incubated at room temperature (25 °C) for 1 hour.

Then, a Desalt Spin Columns^{™} 0.5 ml (25 columns) (Thermo, Catalog No.: 89882) spin column was put into a 2 mL tube and centrifuged at 1500 x g, 4 °C for 1 minute to remove free Cy3. To the column, 300 µL of TBS was added, which was centrifuged at 1500 x g, 4 °C for 1 minute, and this process was repeated three times. The column was put into a new 1.5 mL tube, a labelled sample (100 µL) was added to the column, which was centrifuged at 1500 x g, 4 °C for 2 minutes.

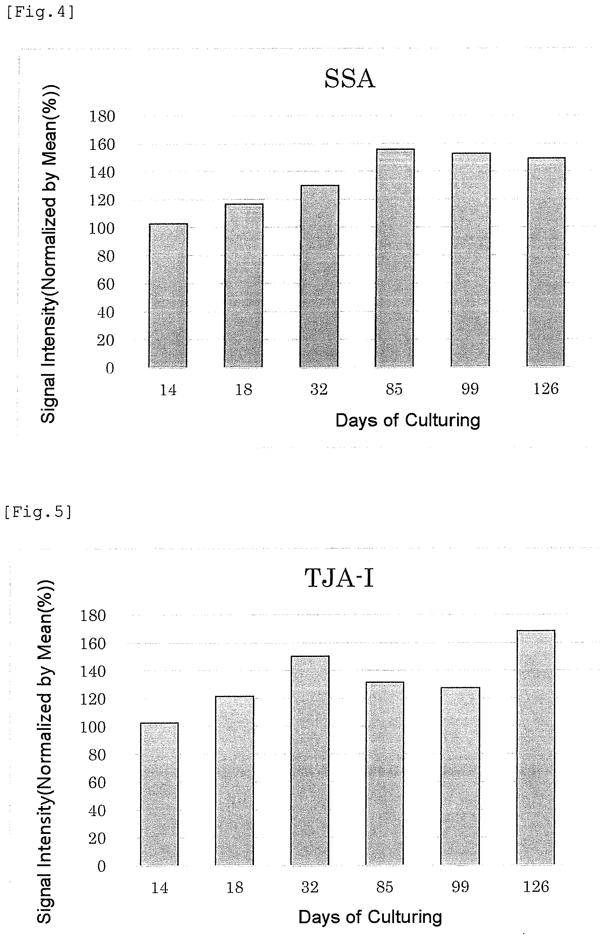

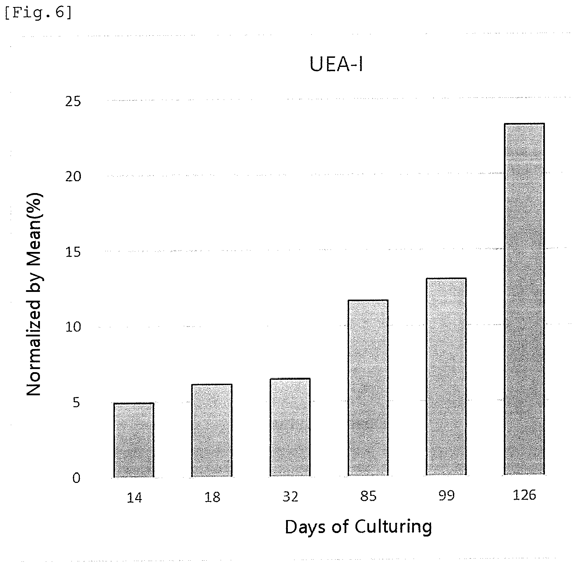

The sample was collected, to which 405 µL of Probing Solution (Glyco Technica) was added to make the sample volume of 500 µL at a concentration of 2 µg/mL. All samples were diluted using Probing Solution to a concentration within a range between 2 µg/mL and 15.625 ng/mL. Then, LecChip Ver.1.0 (trademark, Glyco technica) was washed three times with Probing Solution before adding the sample to the LecChip at 60µL/well. The sample on the LecChip was reacted at 20 °C for 13 hours. The fluorescence pattern of LecChip^{™} was measured four times in total by GlycoLite2200 ((trademark), Glyco technica) with exposure time of 1996, 2995, 3993, 4992, 6988 and 9984 (milliseconds), with fixed camera gain. The obtained 45 lectin signals were measured by GlycoStaion^{®} ToolsPro Suite 1.5 (GlycoStaion^{™} ToolsPro Suite 1.5.), and the measured values were normalized by mean by dividing them by mean intensity of 45 lectin signals and then multiplying by 100 based on A. Kuno et al., J. of Proteomics & Bioinformatics, Vol.1, May 2008, p.68. Table 8 and Figs.3-6 show the results of the signal intensities for SNA, SSA and TJA-I, UEA-1 on cell surface of the cells constituting the chondrocyte culture obtained according to the method above. The numbers 14 to 126 described in the top row of the table indicate the number of days for which the collected samples were cultured.

**[Table 8]**

| | | | | | | |
|---|---|---|---|---|---|---|
| | 14 | 18 | 32 | 85 | 99 | 126 |
| SNA | 89.104343 | 106.46617 | 138.052 | 153.154 | 169.141 | 165.411 |
| SSA | 103.01422 | 116.85575 | 130.017 | 155.771 | 152.932 | 149.318 |
| TJA-I | 102.86368 | 121.68225 | 150.391 | 131.595 | 127.55 | 168.479 |
| UEA-I | 4.903315304 | 6.15704387 | 6.500817 | 11.64882 | 13.03299 | 23.26971 |

| | | | | | | |
|---|---|---|---|---|---|---|
| **(Signal intensity (normalization by mean (%))** | | | | | | |

From Table 8 and Figs. 3-6, it can be seen that, in Working Example 1, the signals of an α2-6 sialic acid-binding lectin such as SNA, SSA and TJA-I and an α1-2 fucose-binding lectin such as UEA-1 are enhanced depending on the number of days of culturing. Alpha2-6 sialic acid which reacts to SNA, SSA and TJA-I is a marker for somatic stem cells such as mesenchymal stem cells (MSCs) or chondrocyte progenitor cells which have high differentiation potential (WO2016/006712A1), whereas the α1-2 fucose-binding lectin such as UEA-1 has been known to be a pluripotent stem cell marker (Wang et al., Cell Res. 2011 Nov;21(11):1551-63. doi:
10.1038/cr.2011.148. Epub 2011 Sep 6.). In the chondrocyte culture produced in Working Example 1, increases in α2-6 sialic acid and α1-2 fucose which react to SNA, SSA, TJA-I and UEA-1 were confirmed along with the days of culturing, indicating that the tissue culture comprises a large number of somatic stem cells and pluripotent stem cells which have high differentiation potential. Accordingly, it became clear that the chondrocyte culture of Working Example 1 is a tissue culture having a high tissue regeneration ability.

### 2. Hyaluronic Acid Retaining Assay for TGP Gel

### [Working Example 2]

Five milligrams of hyaluronic acid (TEIJIN PHARMA Ltd.) was dissolved in 2.5ml of DMEM solution containing antibiotics (gentamicin (50 µg/ml), amphotericin (0.25 µg/ml), penicillin (100 Units/ml)/streptomycin (100 µg/ml)) and L-ascorbic acid (5 mg/ml), which was named Liquid Compositions 1 and 2.

One gram of TGP generated in Production Example 1 was dissolved at °C in 9 ml of DMEM to give 10 % TGP solution, to which hyaluronic acid was added such that the hyaluronic acid concentration would be the same as those of Liquid Compositions 1 and 2, and shaken to uniformize hyaluronic acid in the TGP solution. The TGP solution was gelled at 37 °C, then 1.0 ml of a liquid medium (DMEM) was added thereto to give TGP Composition 1 and TGP Composition 2.

The produced hyaluronic acid-comprising liquid compositions and TGP compositions were put into 5 % carbon dioxide incubator at 37 °C, and the concentration of hyaluronic acid contained in each liquid medium part was measured by the same method as aforementioned <Measuring Hyaluronic Acid>. The results are shown in Table 9.

**[Table 9]**

| Number of days | Liquid Composition 1 | Liquid Composition 2 | TGP Composition 1 | TGP Composition 2 |
|---|---|---|---|---|
| 7 | 12805.8 | 13044.2 | 6.1 | 11.4 |
| 14 | 141.5 | 177.9 | 7.3 | 7.9 |
| 21 | 5.6 | 7.2 | 10.3 | 11.5 |

| | | | | |
|---|---|---|---|---|
| (ng/ml) | | | | |

In Liquid Compositions 1 and 2, there was hyaluronic acid at approximately 13000 ng/ml on Day 7, which was decreased to 5.6 ng/ml on Day 21. On the other hand, TGP Compositions 1 and 2 contained hyaluronic acid at 6.1 and 11.4 ng/ml even on Day 7. These concentrations are as low as approximately 1/1000 of that in Liquid Compositions 1 and 2, indicating that a large part of hyaluronic acid was retained within the TGP gel. Moreover, the concentrations was 10.3 and 11.5 ng/ml on Day 21, showing little change in concentration. Therefore, it became clear that the TGP gel has an ability to stably retain hyaluronic acid without being degraded for a prolonged period.

## Claims

1. A method of producing a chondrocyte culture with high tissue-regenerating ability, the method comprising a step of culturing a cell population separated from cartilage tissue with a thermoreversible gelation polymer.

2. The method according to Claim 1, wherein the tissue-regenerating ability is an ability of the chondrocyte culture to express one or more genes selected from SOX9, COL2A1, miR140 and miR21, an ability to retain miRNAs, stem cell content, or an ability to secret hyaluronic acid.

3. The method according to Claim 2, wherein the miRNA is miR140.

4. The method according to Claim 2 or 3, wherein the stem cell is a pluripotent stem cell or a somatic stem cell with high differentiation potential.

5. The method according to any one of Claims 2 to 4, wherein the stem cell content is the content of 1-2 fucose or α2-6 sialic acid in the chondrocyte culture.

6. The method according to any one of Claims 1 to 5, wherein the cartilage tissue is derived from a subject who is at or more than the age of 50 and has osteoarthritis._{∘}

7. The method according to any one of Claims 1 to 6, wherein the thermoreversible gelation polymer is a thermoreversible gelation polymer with no added growth factor other than serum.

8. A chondrocyte culture produced by the method according to any one of Claims 1 to 7.

9. A method of treating a disease in a subject, comprising applying an effective amount of the chondrocyte culture according to Claim 8 to a subject in need thereof.

10. The method of treating according to Claim 9, wherein the disease is osteoarthritis.


SEQUENCE LISTING

<110>  GN Corporation Ltd\201@and JBM Incorporation

<120>  Chondrocyte culture with high tissue regeneration capacity

<130>  1004GN

<160>  16

<170>  PatentIn version 3.5

<210>  1
<211>  3931
<212>  DNA
<213>  Homo sapiens

<400>  1
ggagagccga aagcggagct cgaaactgac tggaaacttc agtggcgcgg agactcgcca      60

gtttcaaccc cggaaacttt tctttgcagg aggagaagag aaggggtgca agcgccccca     120

cttttgctct ttttcctccc ctcctcctcc tctccaattc gcctcccccc acttggagcg     180

ggcagctgtg aactggccac cccgcgcctt cctaagtgct cgccgcggta gccggccgac     240

gcgccagctt ccccgggagc cgcttgctcc gcatccgggc agccgagggg agaggagccc     300

gcgcctcgag tccccgagcc gccgcggctt ctcgcctttc ccggccacca gccccctgcc     360

ccgggcccgc gtatgaatct cctggacccc ttcatgaaga tgaccgacga gcaggagaag     420

ggcctgtccg gcgcccccag ccccaccatg tccgaggact ccgcgggctc gccctgcccg     480

tcgggctccg gctcggacac cgagaacacg cggccccagg agaacacgtt ccccaagggc     540

gagcccgatc tgaagaagga gagcgaggag gacaagttcc ccgtgtgcat ccgcgaggcg     600

gtcagccagg tgctcaaagg ctacgactgg acgctggtgc ccatgccggt gcgcgtcaac     660

ggctccagca agaacaagcc gcacgtcaag cggcccatga acgccttcat ggtgtgggcg     720

caggcggcgc gcaggaagct cgcggaccag tacccgcact tgcacaacgc cgagctcagc     780

aagacgctgg gcaagctctg gagacttctg aacgagagcg agaagcggcc cttcgtggag     840

gaggcggagc ggctgcgcgt gcagcacaag aaggaccacc cggattacaa gtaccagccg     900

cggcggagga agtcggtgaa gaacgggcag gcggaggcag aggaggccac ggagcagacg     960

cacatctccc ccaacgccat cttcaaggcg ctgcaggccg actcgccaca ctcctcctcc    1020

ggcatgagcg aggtgcactc ccccggcgag cactcggggc aatcccaggg cccaccgacc    1080

ccacccacca cccccaaaac cgacgtgcag ccgggcaagg ctgacctgaa gcgagagggg    1140

cgccccttgc cagagggggg cagacagccc cctatcgact tccgcgacgt ggacatcggc    1200

gagctgagca gcgacgtcat ctccaacatc gagaccttcg atgtcaacga gtttgaccag    1260

tacctgccgc ccaacggcca cccgggggtg ccggccacgc acggccaggt cacctacacg    1320

ggcagctacg gcatcagcag caccgcggcc accccggcga gcgcgggcca cgtgtggatg    1380

tccaagcagc aggcgccgcc gccacccccg cagcagcccc cacaggcccc gccggccccg 1440

caggcgcccc cgcagccgca ggcggcgccc ccacagcagc cggcggcacc cccgcagcag 1500

ccacaggcgc acacgctgac cacgctgagc agcgagccgg gccagtccca gcgaacgcac 1560

atcaagacgg agcagctgag ccccagccac tacagcgagc agcagcagca ctcgccccaa 1620

cagatcgcct acagcccctt caacctccca cactacagcc cctcctaccc gcccatcacc 1680

cgctcacagt acgactacac cgaccaccag aactccagct cctactacag ccacgcggca 1740

ggccagggca ccggcctcta ctccaccttc acctacatga accccgctca gcgccccatg 1800

tacaccccca tcgccgacac ctctggggtc ccttccatcc cgcagaccca cagcccccag 1860

cactgggaac aacccgtcta cacacagctc actcgacctt gaggaggcct cccacgaagg 1920

gcgaagatgg ccgagatgat cctaaaaata accgaagaaa gagaggacca accagaattc 1980

cctttggaca tttgtgtttt tttgtttttt tattttgttt tgttttttct tcttcttctt 2040

cttccttaaa gacatttaag ctaaaggcaa ctcgtaccca aatttccaag acacaaacat 2100

gacctatcca agcgcattac ccacttgtgg ccaatcagtg gccaggccaa ccttggctaa 2160

atggagcagc gaaatcaacg agaaactgga ctttttaaac cctcttcaga gcaagcgtgg 2220

aggatgatgg agaatcgtgt gatcagtgtg ctaaatctct ctgcctgttt ggactttgta 2280

attatttttt tagcagtaat taaagaaaaa agtcctctgt gaggaatatt ctctatttta 2340

aatattttta gtatgtactg tgtatgattc attaccattt tgaggggatt tatacatatt 2400

tttagataaa attaaatgct cttatttttc caacagctaa actactctta gttgaacagt 2460

gtgccctagc ttttcttgca accagagtat ttttgtacag atttgctttc tcttacaaaa 2520

agaaaaaaaa aatcctgttg tattaacatt taaaaacaga attgtgttat gtgatcagtt 2580

ttgggggtta actttgctta attcctcagg ctttgcgatt taaggaggag ctgccttaaa 2640

aaaaaataaa ggccttattt tgcaattatg ggagtaaaca atagtctaga gaagcatttg 2700

gtaagcttta tcatatatat attttttaaa gaagagaaaa acaccttgag ccttaaaacg 2760

gtgctgctgg gaaacatttg cactctttta gtgcatttcc tcctgccttt gcttgttcac 2820

tgcagtctta agaaagaggt aaaaggcaag caaaggagat gaaatctgtt ctgggaatgt 2880

ttcagcagcc aataagtgcc cgagcacact gcccccggtt gcctgcctgg gccccatgtg 2940

gaaggcagat gcctgctcgc tctgtcacct gtgcctctca gaacaccagc agttaacctt 3000

caagacattc cacttgctaa aattatttat tttgtaagga gaggttttaa ttaaaacaaa 3060

aaaaaattct tttttttttt tttttccaat tttaccttct ttaaaatagg ttgttggagc 3120

tttcctcaaa gggtatggtc atctgttgtt aaattatgtt cttaactgta accagttttt 3180

ttttatttat ctctttaatc tttttttatt attaaaagca agtttctttg tattcctcac 3240

cctagatttg tataaatgcc tttttgtcca tccctttttt ctttgttgtt tttgttgaaa 3300

acaaactgga aacttgtttc tttttttgta taaatgagag attgcaaatg tagtgtatca 3360

ctgagtcatt tgcagtgttt tctgccacag acctttgggc tgccttatat tgtgtgtgtg 3420

tgtgggtgtg tgtgtgtttt gacacaaaaa caatgcaagc atgtgtcatc catatttctc 3480

tgcatcttct cttggagtga gggaggctac ctggagggga tcagcccact gacagacctt 3540

aatcttaatt actgctgtgg ctagagagtt tgaggattgc tttttaaaaa agacagcaaa 3600

cttttttttt tatttaaaaa aagatatatt aacagtttta gaagtcagta gaataaaatc 3660

ttaaagcact cataatatgg catccttcaa tttctgtata aaagcagatc tttttaaaaa 3720

gatacttctg taacttaaga aacctggcat ttaaatcata ttttgtcttt aggtaaaagc 3780

tttggtttgt gttcgtgttt tgtttgtttc acttgtttcc ctcccagccc caaacctttt 3840

gttctctccg tgaaacttac ctttcccttt ttctttctct tttttttttt tgtatattat 3900

tgtttacaat aaatatacat tgcattaaaa a 3931

<210> 2
<211> 5059
<212> DNA
<213> Homo sapiens

<400> 2
gcagagcgct gctgggctgc cgggtctccc gcttccccct cctgctccaa gggcctcctg 60

catgagggcg cggtagagac ccggacccgc gccgtgctcc tgccgtttcg ctgcgctccg 120

cccgggcccg gctcagccag gccccgcggt gagccatgat tcgcctcggg gctccccaga 180

cgctggtgct gctgacgctg ctcgtcgccg ctgtccttcg gtgtcagggc caggatgtcc 240

aggaggctgg cagctgtgtg caggatgggc agaggtataa tgataaggat gtgtggaagc 300

cggagccctg ccggatctgt gtctgtgaca ctgggactgt cctctgcgac gacataatct 360

gtgaagacgt gaaagactgc ctcagccctg agatcccctt cggagagtgc tgccccatct 420

gcccaactga cctcgccact gccagtgggc aaccaggacc aaagggacag aaaggagaac 480

ctggagacat caaggatatt gtaggaccca aaggacctcc tgggcctcag ggacctgcag 540

gggaacaagg acccagaggg gatcgtggtg acaaaggtga aaaaggtgcc cctggacctc 600

gtggcagaga tggagaacct gggacccctg gaaatcctgg cccccctggt cctcccggcc 660

cccctggtcc ccctggtctt ggtggaaact ttgctgccca gatggctgga ggatttgatg 720

aaaaggctgg tggcgcccag ttgggagtaa tgcaaggacc aatgggcccc atgggacctc 780

gaggacctcc aggccctgca ggtgctcctg ggcctcaagg atttcaaggc aatcctggtg 840

aacctggtga acctggtgtc tctggtccca tgggtccccg tggtcctcct ggtccccctg 900

gaaagcctgg tgatgatggt gaagctggaa aacctggaaa agctggtgaa aggggtccgc 960

ctggtcctca gggtgctcgt ggtttcccag gaaccccagg ccttcctggt gtcaaaggtc 1020

acagaggtta tccaggcctg gacggtgcta agggagaggc gggtgctcct ggtgtgaagg 1080

gtgagagtgg ttccccgggt gagaacggat ctccgggccc aatgggtcct cgtggcctgc 1140

ctggtgaaag aggacggact ggccctgctg gcgctgcggg tgcccgaggc aacgatggtc 1200

agccaggccc cgcagggcct ccgggtcctg tcggtcctgc tggtggtcct ggcttccctg 1260

gtgctcctgg agccaagggt gaagccggcc ccactggtgc ccgtggtcct gaaggtgctc 1320

aaggtcctcg cggtgaacct ggtactcctg ggtcccctgg gcctgctggt gcctccggta 1380

accctggaac agatggaatt cctggagcca aaggatctgc tggtgctcct ggcattgctg 1440

gtgctcctgg cttccctggg ccacggggcc ctcctggccc tcaaggtgca actggtcctc 1500

tgggcccgaa aggtcagacg ggtgaacctg gtattgctgg cttcaaaggt gaacaaggcc 1560

ccaaggagaa acctggccct gctggccccc agggagcccc tggacccgct ggtgaagaag 1620

gcaagagagg tgcccgtgga gagcctggtg gcgttggccc catcggtccc cctggagaaa 1680

gaggtgctcc cggcaaccgc ggtttcccag gtcaagatgg tctggcaggt cccaagggag 1740

cccctggaga gcgagggccc agtggtcttg ctggccccaa gggagccaac ggtgaccctg 1800

gccgtcctgg agaacctggc cttcctggag cccggggtct cactggccgc cctggtgatg 1860

ctggtcctca aggcaaagtt ggcccttctg gagcccctgg tgaagatggt cgtcctggac 1920

ctccaggtcc tcagggggct cgtgggcagc ctggtgtcat gggtttccct ggccccaaag 1980

gtgccaacgg tgagcctggc aaagctggtg agaagggact gcctggtgct cctggtctga 2040

ggggtcttcc tggcaaagat ggtgagacag gtgctgcagg accccctggc cctgctggac 2100

ctgctggtga acgaggcgag cagggtgctc ctgggccatc tgggttccag ggacttcctg 2160

gccctcctgg tcccccaggt gaaggtggaa aaccaggtga ccagggtgtt cccggtgaag 2220

ctggagcccc tggcctcgtg ggtcccaggg gtgaacgagg tttcccaggt gaacgtggct 2280

ctcccggtgc ccagggcctc cagggtcccc gtggcctccc cggcactcct ggcactgatg 2340

gtcccaaagg tgcatctggc ccagcaggcc cccctggggc tcagggccct ccaggtcttc 2400

agggaatgcc tggcgagagg ggagcagctg gtatcgctgg gcccaaaggc gacaggggtg 2460

acgttggtga gaaaggccct gagggagccc ctggaaagga tggtggacga ggcctgacag 2520

gtcccattgg cccccctggc ccagctggtg ctaatggcga gaagggagaa gttggacctc 2580

ctggtcctgc aggaagtgct ggtgctcgtg gcgctccggg tgaacgtgga gagactgggc 2640

cccccggacc agcgggattt gctgggcctc ctggtgctga tggccagcct ggggccaagg 2700

gtgagcaagg agaggccggc cagaaaggcg atgctggtgc ccctggtcct cagggcccct 2760

ctggagcacc tgggcctcag ggtcctactg gagtgactgg tcctaaagga gcccgaggtg 2820

cccaaggccc cccgggagcc actggattcc ctggagctgc tggccgcgtt ggacccccag 2880

gctccaatgg caaccctgga ccccctggtc cccctggtcc ttctggaaaa gatggtccca 2940

aaggtgctcg aggagacagc ggcccccctg gccgagctgg tgaacccggc ctccaaggtc 3000

ctgctggacc ccctggcgag aagggagagc ctggagatga cggtccctct ggtgccgaag 3060

gtccaccagg tccccagggt ctggctggtc agagaggcat cgtcggtctg cctgggcaac 3120

gtggtgagag aggattccct ggcttgcctg gcccgtcggg tgagcccggc aagcagggtg 3180

ctcctggagc atctggagac agaggtcctc ctggccccgt gggtcctcct ggcctgacgg 3240

gtcctgcagg tgaacctgga cgagagggaa gccccggtgc tgatggcccc cctggcagag 3300

atggcgctgc tggagtcaag ggtgatcgtg gtgagactgg tgctgtggga gctcctggag 3360

cccctgggcc ccctggctcc cctggccccg ctggtccaac tggcaagcaa ggagacagag 3420

gagaagctgg tgcacaaggc cccatgggac cctcaggacc agctggagcc cggggaatcc 3480

agggtcctca aggccccaga ggtgacaaag gagaggctgg agagcctggc gagagaggcc 3540

tgaagggaca ccgtggcttc actggtctgc agggtctgcc cggccctcct ggtccttctg 3600

gagaccaagg tgcttctggt cctgctggtc cttctggccc tagaggtcct cctggccccg 3660

tcggtccctc tggcaaagat ggtgctaatg gaatccctgg ccccattggg cctcctggtc 3720

cccgtggacg atcaggcgaa accggccctg ctggtcctcc tggaaatcct ggaccccctg 3780

gtcctccagg tccccctggc cctggcatcg acatgtccgc ctttgctggc ttaggcccga 3840

gagagaaggg ccccgacccc ctgcagtaca tgcgggccga ccaggcagcc ggtggcctga 3900

gacagcatga cgccgaggtg gatgccacac tcaagtccct caacaaccag attgagagca 3960

tccgcagccc cgagggctcc cgcaagaacc ctgctcgcac ctgcagagac ctgaaactct 4020

gccaccctga gtggaagagt ggagactact ggattgaccc caaccaaggc tgcaccttgg 4080

acgccatgaa ggttttctgc aacatggaga ctggcgagac ttgcgtctac cccaatccag 4140

caaacgttcc caagaagaac tggtggagca gcaagagcaa ggagaagaaa cacatctggt 4200

ttggagaaac catcaatggt ggcttccatt tcagctatgg agatgacaat ctggctccca 4260

acactgccaa cgtccagatg accttcctac gcctgctgtc cacggaaggc tcccagaaca 4320

tcacctacca ctgcaagaac agcattgcct atctggacga agcagctggc aacctcaaga 4380

aggccctgct catccagggc tccaatgacg tggagatccg ggcagagggc aatagcaggt 4440

tcacgtacac tgccctgaag gatggctgca cgaaacatac cggtaagtgg ggcaagactg 4500

ttatcgagta ccggtcacag aagacctcac gcctccccat cattgacatt gcacccatgg 4560

acataggagg gcccgagcag gaattcggtg tggacatagg gccggtctgc ttcttgtaaa 4620

aacctgaacc cagaaacaac acaatccgtt gcaaacccaa aggacccaag tactttccaa 4680

tctcagtcac tctaggactc tgcactgaat ggctgacctg acctgatgtc cattcatccc 4740

accctctcac agttcggact tttctcccct ctctttctaa gagacctgaa ctgggcagac 4800

tgcaaaataa aatctcggtg ttctatttat ttattgtctt cctgtaagac cttcgggtca 4860

aggcagaggc aggaaactaa ctggtgtgag tcaaatgccc cctgagtgac tgcccccagc 4920

ccaggccaga agacctccct tcaggtgccg ggcgcaggaa ctgtgtgtgt cctacacaat 4980

ggtgctattc tgtgtcaaac acctctgtat tttttaaaac atcaattgat attaaaaatg 5040

aaaagattat tggaaagta 5059

<210> 3
<211> 100
<212> RNA
<213> Homo sapiens

<400> 3
ugugucucuc ucuguguccu gccagugguu uuacccuaug guagguuacg ucaugcuguu 60

cuaccacagg guagaaccac ggacaggaua ccggggcacc 100

<210> 4
<211> 21
<212> RNA
<213> Homo sapiens

<400> 4
uaccacaggg uagaaccacg g 21

<210> 5
<211> 22
<212> RNA
<213> Homo sapiens

<400> 5
cagugguuuu acccuauggu ag 22

<210> 6
<211> 72
<212> RNA
<213> Homo sapiens

<400> 6
ugucggguag cuuaucagac ugauguugac uguugaaucu cauggcaaca ccagucgaug 60

ggcugucuga ca 72

<210> 7
<211> 21
<212> RNA
<213> Homo sapiens

<400> 7
caacaccagu cgaugggcug u 21

<210> 8

<211>  22
<212>  RNA
<213>  Homo sapiens

<400>  8
uagcuuauca gacugauguu ga                                                22


<210>  9
<211>  20
<212>  DNA
<213>  Artificial Sequence

<220>
<223>  Primer Fwd

<400>  9
caacaccagt cgatggctgt                                                   20


<210>  10
<211>  22
<212>  DNA
<213>  Artificial Sequence

<220>
<223>  Primer Fwd

<400>  10
tagcttatca gactgatgtt ga                                                22


<210>  11
<211>  21
<212>  DNA
<213>  Artificial Sequence

<220>
<223>  Primer Fwd

<400>  11
cagtggtttt accctatggt a                                                 21


<210>  12
<211>  21
<212>  DNA
<213>  Artificial Sequence

<220>
<223>  Primer Fwd

<400>  12
taccacaggg tagaaccacg g                                                 21


<210>  13
<211>  25
<212>  DNA
<213>  Artificial Sequence

<220>
<223>  Primer Fwd

<400>  13
ggagatgaaa tctgttctgg gaatg                                              25

<210>  14
<211>  25
<212>  DNA
<213>  Artificial Sequence

<220>
<223>  Primer Rev

<400>  14
ttgaaggtta actgctggtg ttctg                                              25

<210>  15
<211>  22
<212>  DNA
<213>  Artificial Sequence

<220>
<223>  Primer Fwd

<400>  15
ccagttggga gtaatgcaag ga                                                 22

<210>  16
<211>  21
<212>  DNA
<213>  Artificial Sequence

<220>
<223>  Primer Rev

<400>  16
acaccaggtt caccaggttc a                                                  21